# EUROPEAN PATENT APPLICATION

(11) **EP 2 377 552 A2**
(43) Date of publication of application: **19.10.2011**
(21) Application number: 11162696.6
(22) Date of filing: 06.11.2006
(51) Int. Cl.: A61K 39/39, A61K 39/145

(54) **Influenza vaccines with reduced amount of emulsion adjuvant**

(30) Priority: 04.11.2005 US 734026 P; 05.01.2006 US 757058 P; 19.05.2006 US 801680 P
(62) Divisional of application: 06831740.3
(71) Applicant: Novartis Vaccines and Diagnostics S.r.l., 53100 Siena (SI) (IT)
(72) Inventor: Contorni, Mario, 53100 Siena (IT)
(74) Representative: Carpmaels & Ransford

(57) **Abstract**

Influenza vaccines with oil-in-water emulsion adjuvants are known. The amount of emulsion adjuvant required for an influenza vaccine can be reduced, thereby allowing more vaccines to be made from a given amount of emulsion, and/or minimizing the amount of emulsion that has to be produced for a given number of vaccine doses. These vaccines can conveniently be made by mixing (i) an oil-in-water emulsion and (ii) an aqueous preparation of an influenza virus antigen. In one aspect, substantially equal volumes of components (i) and (ii) are used; in another aspect, an excess volume of component (ii) is used. When using substantially equal volumes, component (ii) has a hemagglutinin concentration of more than 60µg per influenza virus strain per ml. Components (i) and (ii) can be presented in kit form.

## Description

All documents cited herein are incorporated by reference in their entirety.

### TECHNICAL FIELD

This invention is in the field of adjuvanted vaccines for protecting against influenza virus infection.

### BACKGROUND ART

Influenza vaccines currently in general use do not include an adjuvant. These vaccines are described in more detail in chapters 17 & 18 of reference 1. They are based on live virus or inactivated virus, and inactivated vaccines can be based on whole virus, 'split' virus or on purified surface antigens (including haemagglutinin and neuraminidase). Haemagglutinin (HA) is the main immunogen in inactivated influenza vaccines, and vaccine doses are standardized by reference to HA levels, with vaccines typically containing about 15µg of HA per strain.

In a pandemic influenza outbreak then a large number of doses of influenza vaccine will be needed, but it will be difficult to increase vaccine supply to meet the huge demand. Rather than produce more vaccine antigen, therefore, it has been proposed to use a lower amount of antigen per strain, and to use an adjuvant to compensate for the reduced antigen dose. It has also been proposed to use the same approach in inter-pandemic periods e.g. to allow greater coverage of the population without increasing manufacturing levels.

One adjuvanted vaccine is already available, namely the FLUAD^{TM} product. The adjuvant in this vaccine is an oil-in-water emulsion. In the FLUAD^{TM} product, the antigen and the emulsion adjuvant are supplied in pre-mixed format in a pre-filled syringe. The product datasheet shows that each dose has a volume of 0.5ml and contains 15µg HA per strain, 9.75mg squalene, 1.175 mg polysorbate 80, 1.175mg sorbitan trioleate, 0.66mg sodium citrate, 0.04mg citric acid, and water for injection. As disclosed in reference 2, the vaccine is made by mixing at a 2X emulsion with a 2X antigen solution at a 1:1 volumetric ratio, to give a final solution with both emulsion and antigen at the 1X concentration. This 1:1 mixing ratio is further explained in chapter 10 of reference 8.

It is an object of the invention to provide further and improved influenza vaccines with oil-in-water adjuvants (for both pandemic and interpandemic use) and methods for their preparation.

### DISCLOSURE OF THE INVENTION

In addition to the FLUAD^{TM} product, experimental MF59-adjuvanted influenza vaccines have been tested in humans, including vaccines with reduced antigen doses. In references 3 & 4, for instance, a MF59-adjuvanted vaccine based on a H5N3 strain was tested in which HA was present either at the normal dose (15µg), a double dose (30µg) or a half dose (7.5µg). In all cases, however, the patients received a 0.5ml volume of pre-mixed vaccine containing a full amount (1X) of MF59 adjuvant.

The inventor has now realized that a full amount of oil-in-water emulsion adjuvant may not always be necessary. According to the invention, therefore, the amount of emulsion adjuvant required for an influenza vaccine is reduced, thereby allowing more vaccines to be made from a given amount of emulsion, and/or minimizing the amount of emulsion that has to be produced for a given number of vaccine doses. In a situation where many doses are required very quickly then a reduction in overall requirements for emulsion adjuvants will be advantageous.

These vaccines can conveniently be made, as already known in the art, by mixing (i) an oil-in-water emulsion and (ii) an aqueous preparation of an influenza virus antigen. With the invention, however, the mixing procedure can differ from the prior art in various ways, and the choice of procedure can have various effects on dose and volume when compared to the FLUAD^{TM} product. For example:
● As in the prior art, substantially equal volumes of components (i) and (ii) can be used. Unlike the prior art, however, by using less than 0.25ml of emulsion then the volume required for making the final vaccine can be reduced. If the antigen concentration in component (ii) is increased to match the volume decrease then the amount of antigen in the final vaccine can be maintained (15µg per strain); if the antigen concentration in component (ii) is maintained (15µg per strain per 0.25ml) then the final vaccine will have a reduced HA dose.
● Unlike the prior art, an excess volume of component (ii) can be used. If the antigen concentration is decreased to match the increase in excess then the amount of antigen in the final vaccine can be maintained (15µg per strain); if the antigen concentration is decreased more than the increase in excess then the final antigen dose decreases (<15µg per strain); and if the antigen concentration is maintained (15µg per strain per 0.25ml) then the final vaccine will have an increased HA dose (>15µg per strain).

Thus the invention provides a process for making an adjuvanted influenza vaccine, comprising a step of mixing substantially equal volumes of (i) an oil-in-water emulsion and (ii) an aqueous preparation of an influenza virus antigen, wherein the concentration of hemagglutinin in component (ii) is more than 60µg per influenza virus strain per ml. By mixing equal volumes then the HA concentration after mixing will be >30µg per influenza virus strain per ml. Thus this process gives a composition in which the final HA concentration in a 0.5ml volume is >15µg/strain, and so a dose of 15µg/strain can be achieved by administering <0.5ml of this vaccine.

The invention also provides a process for making an adjuvanted influenza vaccine, comprising a step of mixing substantially equal volumes of (i) an oil-in-water emulsion and (ii) an aqueous preparation of an influenza virus antigen, wherein the vaccine has a volume of less than 0.5ml. The volume may be between 0.05ml and 0.45ml, between 0,1ml and 0.4ml, between 0.2ml and 0.3ml, *etc.* Thus the volume may be about 0.1ml, about 0.15ml, about 0.2ml, about 0.25ml, about 0.3ml, about 0.35ml, about 0.4ml, or about 0.45ml, *etc.* If the concentration of hemagglutinin in component (ii) is about 60µg per influenza virus strain per ml then the vaccine will have a final HA concentration of about 30gg per influenza virus strain per ml and so, with a volume of<0.5ml, the HA dose will be less than 15µg/dose/strain. Adjuvant and antigen requirements are thus reduced.

The invention also provides a process for making an adjuvanted influenza vaccine, comprising the steps of: (a) mixing substantially equal volumes of (i) an oil-in-water emulsion and (ii) an aqueous preparation of an influenza virus antigen, to give a bulk vaccine; and (b) removing at least one unit dose from the bulk vaccine, where the volume of the unit dose is, as described above, <0.5ml.

Thus the invention also provides a process for making an adjuvanted influenza vaccine, comprising a step of mixing substantially equal volumes of (i) an oil-in-water emulsion and (ii) an aqueous preparation of an influenza virus antigen, wherein the concentration of hemagglutinin in the vaccine is less than 30µg per influenza virus strain per ml. Thus this process gives a composition in which the final HA concentration in a standard 0.5ml volume is <15µg/strain. The vaccine may be administered in doses of about 0.5ml or, as described above, <0.5ml.

The invention also provides a process for making an adjuvanted influenza vaccine, comprising the step of mixing a first volume of an oil-in-water emulsion with a second volume of an aqueous preparation of an influenza virus antigen, wherein the second volume is greater than the first volume. In some embodiments, the concentration of hemagglutinin in the second volume is less than 60µg per influenza virus strain per ml.

These processes can be performed during bulk manufacture, to give a pre-mixed vaccine that can be distributed for administration to patients. As an alternative, they may be performed at the point of use, to give an extemporaneous preparation for patient administration. In the latter case then the invention provides a kit comprising: (i) an adjuvant component, comprising an oil-in-water emulsion; and (ii) an antigen component, comprising an aqueous preparation of an influenza virus antigen. Component (i) does not include an influenza virus antigen, and component (ii) is not an oil-in-water emulsion. The kit components may have substantially equal volumes, in which case the concentration of hemagglutinin in component (ii) may be more than 60µg per influenza virus strain per ml. As an alternative, kit component (ii) may have a greater volume than component (i), and preferably has a hemagglutinin concentration of less than 60µg per influenza virus strain per ml. In some embodiments the two kit components may each have volumes of less than 0.25ml, to give a final volume (as described above) after 1:1 mixing of less than 0.5ml.

The invention also provides the compositions obtainable by the processes of the invention. These compositions include influenza virus antigen in admixture with an oil-in-water emulsion. The HA concentration in these compositions may be more than 30µg per strain per ml e.g. the invention provides a composition comprising an oil-in-water emulsion and an influenza virus antigen, wherein the composition provides 15µg of HA per influenza virus strain per dose, with a dose volume of less than 0.5ml.

The invention also provides a vaccine composition comprising an oil-in-water emulsion and influenza virus antigen, wherein the vaccine has a volume (as described above) of less than 0.5ml. The invention also provides a vaccine composition comprising influenza virus haemagglutinin and squalene, wherein the weight ratio of squalene to haemagglutinin is between 50 and 2000 (e.g. between 100 and 1000), and wherein the vaccine has a volume (as described above) of less than 0.5ml. The HA concentration in the vaccine may be >30µg per strain per ml.

The invention also provides a composition comprising influenza virus haemagglutinin and squalene, wherein the weight ratio of squalene to haemagglutinin is between 50 and 2000 (e.g. between 100 and 1000), and wherein the HA concentration is less than 30µg per strain per ml. A unit dose of this composition for immunization may be about 0.5ml or, as described above, may be less than 0.5ml.

The invention also provides a composition comprising an oil-in-water emulsion and hemagglutinin from *n* influenza virus strains, wherein the weight ratio of oil to hemagglutinin is less than 640/n. The ratio may be, for example, ≤600/n, ≤550/n, ≤500/n, ≤450/n, ≤400/n, ≤350/n, ≤300/n, ≤250/n, ≤200/n, ≤150/n, ≤100/n, ≤50/n, *etc.* The value of *n* is preferably 1, 2, 3 or 4. The oil preferably comprises squalene. A unit dose of this composition for immunization may be about 0.5ml or, as described above, may be less than 0.5ml.

The invention also provides a process for making an adjuvanted monovalent influenza vaccine, comprising the step of mixing substantially equal volumes of (i) an oil-in-water emulsion and (ii) an aqueous preparation of an influenza virus antigen from a single strain, wherein said equal volumes are less than 0.22ml (e.g. ≤0.21ml, ≤0.20ml, ≤0.19ml, ≤0.18ml, ≤0.16ml, ≤0.15ml, ≤0.14ml, ≤0.13ml, ≤0.12ml, ≤0.11ml, ≤0.10ml, ≤0.09ml, ≤0.08ml, ≤0.07ml , ≤0.06ml , ≤0.05ml, *etc.).* Corresponding kits are also provided. These monovalent compositions and kits are particularly useful with pandemic influenza strains (see below).

The invention also provides a process for making an adjuvanted monovalent influenza vaccine, comprising the step of mixing a first volume of an oil-in-water emulsion with a second volume of an aqueous preparation of an influenza virus antigen from a single strain, wherein the second volume is greater than the first volume. In some embodiments, the concentration of haemagglutinin in the second volume is less than 60µg/ml.

The invention also provides a process for making an adjuvanted monovalent influenza vaccine, comprising a step of mixing substantially equal volumes of (i) an oil-in-water emulsion and (ii) an aqueous preparation of an influenza virus antigen from a single strain, wherein the emulsion has a squalene concentration of less than 38mg/ml. Thus the squalene concentration after mixing will be less than 19mg/ml. Corresponding kits are also provided, in which case the equal volumes are preferably about 0.25ml. These monovalent compositions and kits are particularly useful with pandemic influenza strains (see below). The squalene concentration in component (i) may be, for example, ≤37mg/ml, ≤36mg/ml, ≤35mg/ml, ≤34mg/ml, ≤33mg/ml, ≤32mg/ml, ≤31mg/ml, ≤30mg/ml, ≤29mg/ml, ≤28mg/ml, ≤27mg/ml, ≤26mg/ml, ≤25mg/ml, ≤24mg/ml, ≤23mg/ml, ≤22mg/ml, ≤21mg/ml, ≤20mg/ml, ≤19mg/ml, ≤18mg/ml, ≤17mg/ml, ≤16mg/ml, ≤15mg/ml, ≤14mg/ml, ≤13mg/ml, ≤12mg/ml, ≤11mg/ml, ≤10mg/ml, ≤9mg/ml, ≤8mg/ml, ≤7mg/ml, ≤6mg/ml, ≤5mg/ml, *etc.*

### The oil-in-water emulsion adjuvant

Oil-in-water emulsions have been found to be particularly suitable for use in adjuvanting influenza virus vaccines. Various such emulsions are known, and they typically include at least one oil and at least one surfactant, with the oil(s) and surfactant(s) being biodegradable (metabolisable) and biocompatible. The oil droplets in the emulsion are generally less than 5µm in diameter, and may even have a sub-micron diameter, with these small sizes being achieved with a microfluidiser to provide stable emulsions. Droplets with a size less than 220nm are preferred as they can be subjected to filter sterilization.

The invention can be used with oils such as those from an animal (such as fish) or vegetable source. Sources for vegetable oils include nuts, seeds and grains. Peanut oil, soybean oil, coconut oil, and olive oil, the most commonly available, exemplify the nut oils. Jojoba oil can be used e.g. obtained from the jojoba bean. Seed oils include safflower oil, cottonseed oil, sunflower seed oil, sesame seed oil and the like. In the grain group, corn oil is the most readily available, but the oil of other cereal grains such as wheat, oats, rye, rice, teff, triticale and the like may also be used. 6-10 carbon fatty acid esters of glycerol and 1,2-propanediol, while not occurring naturally in seed oils, may be prepared by hydrolysis, separation and esterification of the appropriate materials starting from the nut and seed oils. Fats and oils from mammalian milk are metabolizable and may therefore be used in the practice of this invention. The procedures for separation, purification, saponification and other means necessary for obtaining pure oils from animal sources are well known in the art. Most fish contain metabolizable oils which may be readily recovered. For example, cod liver oil, shark liver oils, and whale oil such as spermaceti exemplify several of the fish oils which may be used herein. A number of branched chain oils are synthesized biochemically in 5-carbon isoprene units and are generally referred to as terpenoids. Shark liver oil contains a branched, unsaturated terpenoids known as squalene, 2,6,10,15,19,23-hexamethyl-2,6,10,14,18,22-tetracosahexaene, which is particularly preferred herein. Squalane, the saturated analog to squalene, is also a preferred oil. Fish oils, including squalene and squalane, are readily available from commercial sources or may be obtained by methods known in the art. Other preferred oils are the tocopherols (see below). Mixtures of oils can be used.

Surfactants can be classified by their 'HLB' (hydrophile/lipophile balance). Preferred surfactants of the invention have a HLB of at least 10, preferably at least 15, and more preferably at least 16. The invention can be used with surfactants including, but not limited to: the polyoxyethylene sorbitan esters surfactants (commonly referred to as the Tweens), especially polysorbate 20 and polysorbate 80; copolymers of ethylene oxide (EO), propylene oxide (PO), and/or butylene oxide (BO), sold under the DOWFAX^{TM} tradename, such as linear EO/PO block copolymers; octoxynols, which can vary in the number of repeating ethoxy (oxy-1,2-ethanediyl) groups, with octoxynol-9 (Triton X-100, or t-octylphenoxypolyethoxyethanol) being of particular interest; (octylphenoxy)polyethoxyethanol (IGEPAL CA-630/NP-40); phospholipids such as phosphatidylcholine (lecithin); nonylphenol ethoxylates, such as the Tergitol^{TM} NP series; polyoxyethylene fatty ethers derived from lauryl, cetyl, stearyl and oleyl alcohols (known as Brij surfactants), such as triethyleneglycol monolauryl ether (Brij 30); polyoxyethylene-9-lauryl ether; and sorbitan esters (commonly known as the SPANs), such as sorbitan trioleate (Span 85) and sorbitan monolaurate. Preferred surfactants for including in the emulsion are Tween 80 (polyoxyethylene sorbitan monooleate), Span 85 (sorbitan trioleate), lecithin and Triton X-100.

Mixtures of surfactants can be used e.g. Tween 80/Span 85 mixtures, or Tween80/Triton-X100 mixtures. A combination of a polyoxyethylene sorbitan ester such as polyoxyethylene sorbitan monooleate (Tween 80) and an octoxynol such as t-octylphenoxypolyethoxyethanol (Triton X-100) is also suitable. Another useful combination comprises laureth 9 plus a polyoxyethylene sorbitan ester and/or an octoxynol.

Preferred amounts of surfactants (% by weight) are: polyoxyethylene sorbitan esters (such as Tween 80) 0.01 to 1%, in particular about 0.1 %; octyl- or nonylphenoxy polyoxyethanols (such as Triton X-100, or other detergents in the Triton series) 0.001 to 0.1 %, in particular 0.005 to 0.02%; polyoxyethylene ethers (such as laureth 9) 0.1 to 20 %, preferably 0.1 to 10 % and in particular 0.1 to 1 % or about 0.5%.

Specific oil-in-water emulsion adjuvants useful with the invention include, but are not limited to:
- A submicron emulsion of squalene, Tween 80, and Span 85. The composition of the emulsion by volume can be about 5% squalene, about 0.5% polysorbate 80 and about 0.5% Span 85. In weight terms, these ratios become 4.3% squalene, 0.5% polysorbate 80 and 0.48% Span 85. This adjuvant is known as 'MF59' [5-7], as described in more detail in Chapter 10 of ref. 8 and chapter 12 of ref. 9. The MF59 emulsion advantageously includes citrate ions e.g. 10mM sodium citrate buffer.
- An emulsion of squalene, a tocopherol, and Tween 80. The emulsion may include phosphate buffered saline. It may also include Span 85 (e.g. at 1 %) and/or lecithin. These emulsions may have from 2 to 10% squalene, from 2 to 10% tocopherol and from 0.3 to 3% Tween 80, and the weight ratio of squalene:tocopherol is preferably ≤1 as this provides a more stable emulsion. Squalene and Tween 80 may be present volume ratio of about 5:2. One such emulsion can be made by dissolving Tween 80 in PBS to give a 2% solution, then mixing 90ml of this solution with a mixture of(5g of DL-α-tocopherol and 5ml squalene), then microfluidising the mixture. The resulting emulsion may have submicron oil droplets e.g. with an average diameter of between 100 and 250nm, preferably about 180nm.
- An emulsion of squalene, a tocopherol, and a Triton detergent (e.g. Triton X-100). The emulsion may also include a 3d-MPL (see below). The emulsion may contain a phosphate buffer.
- An emulsion comprising a polysorbate (e.g. polysorbate 80), a Triton detergent (e.g. Triton X-100) and a tocopherol (e.g. an α-tocopherol succinate). The emulsion may include these three components at a mass ratio of about 75:11:10 (e.g. 750µg/ml polysorbate 80, 110µg/ml Triton X-100 and 100µg/ml α-tocopherol succinate), and these concentrations should include any contribution of these components from antigens. The emulsion may also include squalene. The emulsion may also include a 3d-MPL (see below). The aqueous phase may contain a phosphate buffer.
- An emulsion of squalane, polysorbate 80 and poloxamer 401 ("Pluronic^{TM} L121"). The emulsion can be formulated in phosphate buffered saline, pH 7.4. This emulsion is a useful delivery vehicle for muramyl dipeptides, and has been used with threonyl-MDP in the "SAF-1" adjuvant [10] (0.05-1% Thr-MDP, 5% squalane, 2.5% Pluronic L121 and 0.2% polysorbate 80). It can also be used without the Thr-MDP, as in the "AF" adjuvant [11] (5% squalane, 1.25% Pluronic L121 and 0.2% polysorbate 80). Microfluidisation is preferred.
- An emulsion having from 0.5-50% of an oil, 0.1-10% of a phospholipid, and 0.05-5% of a non-ionic surfactant. As described in reference 12, preferred phospholipid components are phosphatidylcholine, phosphatidylethanolamine, phosphatidylserine, phosphatidylinositol, phosphatidylglycerol, phosphatidic acid, sphingomyelin and cardiolipin. Submicron droplet sizes are advantageous.
- A submicron oil-in-water emulsion of a non-metabolisable oil (such as light mineral oil) and at least one surfactant (such as lecithin, Tween 80 or Span 80). Additives may be included, such as QuilA saponin, cholesterol, a saponin-lipophile conjugate (such as GPI-0100, described in reference 13, produced by addition of aliphatic amine to desacylsaponin via the carboxyl group of glucuronic acid), dimethyidioctadecylammonium bromide and/or N,N-dioctadecyl-N,N-bis (2-hydroxyethyl)propanediamine.
- An emulsion in which a saponin (e.g. QuilA or QS21) and a sterol *(e.g.* a cholesterol) are associated as helical micelles [14].
- An emulsion comprising a mineral oil, a non-ionic lipophilic ethoxylated fatty alcohol, and a non-ionic hydrophilic surfactant (e.g. an ethoxylated fatty alcohol and/or polyoxyethylene-polyoxypropylene block copolymer) [15].
- An emulsion comprising a mineral oil, a non-ionic hydrophilic ethoxylated fatty alcohol, and a non-ionic lipophilic surfactant (e.g. an ethoxylated fatty alcohol and/or polyoxyethylene-polyoxypropylene block copolymer) [15].

The emulsions are preferably mixed with antigen extemporaneously, at the time of delivery. Thus the adjuvant and antigen are typically kept separately in a packaged or distributed vaccine, ready for final formulation at the time of use. The antigen will generally be in an aqueous form, such that the vaccine is finally prepared by mixing two liquids. Where concentrations of components are given in the above descriptions of specific emulsions, these concentrations are typically for an undiluted composition, and the concentration after mixing with an antigen solution will thus decrease.

After the antigen and adjuvant have been mixed, haemagglutinin antigen will generally remain in aqueous solution but may distribute itself around the oil/water interface. In general, little if any haemagglutinin will enter the oil phase of the emulsion.

Where a composition includes a tocopherol, any of the α, β, γ, δ, ε or ξ tocopherols can be used, but α-tocopherols are preferred. The tocopherol can take several forms e.g. different salts and/or isomers. Salts include organic salts, such as succinate, acetate, nicotinate, etc. D-α-tocopherol and DL-α-tocopherol can both be used. Tocopherols are advantageously included in vaccines for use in elderly patients (e.g. aged 60 years or older) because vitamin E has been reported to have a positive effect on the immune response in this patient group [16]. They also have antioxidant properties that may help to stabilize the emulsions [17]. A preferred α-tocopherol is DL-α-tocopherol, and the preferred salt of this tocopherol is the succinate. The succinate salt has been found to cooperate with TNF-related ligands *in vivo.* Moreover, α-tocopherol succinate is known to be compatible with influenza vaccines and to be a useful preservative as an alternative to mercurial compounds [23]. Preservative-free vaccines are particularly preferred.

### The influenza virus antigen

Compositions of the invention include an influenza virus antigen. The antigen will typically be prepared from influenza virions but, as an alternative, antigens such as haemagglutinin can be expressed in a recombinant host (e.g. in an insect cell line using a baculovirus vector) and used in purified form [18,19]. In general, however, antigens will be from virions.

The antigen may take the form of a live virus or, more preferably, an inactivated virus. Chemical means for inactivating a virus include treatment with an effective amount of one or more of the following agents: detergents, formaldehyde, formalin, (β-propiolactone, or UV light. Additional chemical means for inactivation include treatment with methylene blue, psoralen, carboxyfullerene (C60) or a combination of any thereof. Other methods of viral inactivation are known in the art, such as for example binary ethylamine, acetyl ethyleneimine, or gamma irradiation. The INFLEXAL^{TM} product is a whole virion inactivated vaccine.

Where an inactivated virus is used, the vaccine may comprise whole virion, split virion, or purified surface antigens (including hemagglutinin and, usually, also including neuraminidase).

Virions can be harvested from virus-containing fluids by various methods. For example, a purification process may involve zonal centrifugation using a linear sucrose gradient solution that includes detergent to disrupt the virions. Antigens may then be purified, after optional dilution, by diafiltration.

Split virions are obtained by treating virions with detergents (e.g. ethyl ether, polysorbate 80, deoxycholate, tri-N butyl phosphate, Triton X-100, Triton N101, cetyltrimethylammonium bromide, Tergitol NP9, *etc.)* to produce subvirion preparations, including the `Tween-ether' splitting process. Methods of splitting influenza viruses are well known in the art *e.g.* see refs. 20-25, *etc.* Splitting of the virus is typically carried out by disrupting or fragmenting whole virus, whether infectious or non-infectious with a disrupting concentration of a splitting agent. The disruption results in a full or partial solubilisation of the virus proteins, altering the integrity of the virus. Preferred splitting agents are non-ionic and ionic (e.g. cationic) surfactants e.g. alkylglycosides, alkylthioglycosides, acyl sugars, sulphobetaines, betains, polyoxyethylenealkylethers, N,N-dialkyl-Glucamides, Hecameg, alkylphenoxy-polyethoxyethanols, quaternary ammonium compounds, sarcosyl, CTABs (cetyl trimethyl ammonium bromides), tri-N-butyl phosphate, Cetavlon, myristyltrimethylammonium salts, lipofectin, lipofectamine, and DOT-MA, the octyl- or nonylphenoxy polyoxyethanols (e.g. the Triton surfactants, such as Triton X-100 or Triton N101), polyoxyethylene sorbitan esters (the Tween surfactants), polyoxyethylene ethers, polyoxyethlene esters, *etc.* One useful splitting procedure uses the consecutive effects of sodium deoxycholate and formaldehyde, and splitting can take place during initial virion purification (e.g. in a sucrose density gradient solution). Split virions can usefully be resuspended in sodium phosphate-buffered isotonic sodium chloride solution. The BEGRIVAC^{TM} , FLUARIX^{TM}, FLUZONE^{TM} and FLUSHIELD^{TM} products are split vaccines.

Purified surface antigen vaccines comprise the influenza surface antigens haemagglutinin and, typically, also neuraminidase. Processes for preparing these proteins in purified form are well known in the art. The FLUVIRIN^{TM}, AGRIPPAL^{TM} and INFLUVAC^{TM} products are subunit vaccines.

Influenza antigens can also be presented in the form of virosomes [26] (nucleic acid free viral-like liposomal particles), as in the INFLEXAL V^{TM} and INVAVAC^{TM} products.

The influenza virus may be attenuated. The influenza virus may be temperature-sensitive. The influenza virus may be cold-adapted. These three possibilities apply in particular for live viruses.

Influenza virus strains for use in vaccines change from season to season. In the current inter-pandemic period, vaccines typically include two influenza A strains (H1N1 and H3N2) and one influenza B strain, and trivalent vaccines are typical. The invention may also use viruses from pandemic strains (i. e. strains to which the vaccine recipient and the general human population are immunologically naïve), such as H2, H5, H7 or H9 subtype strains (in particular of influenza A virus), and influenza vaccines for pandemic strains may be monovalent or may be based on a normal trivalent vaccine supplemented by a pandemic strain. Depending on the season and on the nature of the antigen included in the vaccine, however, the invention may protect against one or more of influenza A virus HA subtypes H1, H2, H3, H4, H5, H6, H7, H8, H9, H10, H11, H12, H13, H14, H15 or H16. The invention may protect against one or more of influenza A virus NA subtypes N1, N2, N3, N4, N5, N6, N7, N8 or N9.

Other strains that can usefully be included in the compositions are strains which are resistant to antiviral therapy (e.g. resistant to oseltamivir [27] and/or zanamivir), including resistant pandemic strains [28].

The adjuvanted compositions of the invention are particularly useful for immunizing against pandemic strains. The characteristics of an influenza strain that give it the potential to cause a pandemic outbreak are: (a) it contains a new hemagglutinin compared to the hemagglutinins in currently-circulating human strains, i.e. one that has not been evident in the human population for over a decade *(e.g.* H2), or has not previously been seen at all in the human population (e.g. H5, H6 or H9, that have generally been found only in bird populations), such that the human population will be immunologically naïve to the strain's hemagglutinin; (b) it is capable of being transmitted horizontally in the human population; and (c) it is pathogenic to humans. A virus with H5 haemagglutinin type is preferred for immunising against pandemic influenza, such as a H5N1 strain. Other possible strains include H5N3, H9N2, H2N2, H7N1 and H7N7, and any other emerging potentially pandemic strains. Within the H5 subtype, a virus may fall into HA clade 1, HA clade 1', HA clade 2 or HA clade 3 [29], with clades 1 and 3 being particularly relevant.

Compositions of the invention may include antigen(s) from one or more (e.g. 1, 2, 3, 4 or more) influenza virus strains, including influenza A virus and/or influenza B virus. Where a vaccine includes more than one strain of influenza, the different strains are typically grown separately and are mixed after the viruses have been harvested and antigens have been prepared. Thus a process of the invention may include the step of mixing antigens from more than one influenza strain. A trivalent vaccine is preferred, including antigens from two influenza A virus strains and one influenza B virus strain. In some embodiments of the invention, the compositions may include antigen from a single influenza A strain. In some embodiments, the compositions may include antigen from two influenza A strains, provided that these two strains are not H1N1 and H3N2. In some embodiments, the compositions may include antigen from more than two influenza A strains.

The influenza virus may be a reassortant strain, and may have been obtained by reverse genetics techniques. Reverse genetics techniques [e.g. 30-34] allow influenza viruses with desired genome segments to be prepared *in vitro* using plasmids. Typically, it involves expressing (a) DNA molecules that encode desired viral RNA molecules e.g. from poll promoters, and (b) DNA molecules that encode viral proteins e.g. from po1ll promoters, such that expression of both types of DNA in a cell leads to assembly of a complete intact infectious virion. The DNA preferably provides all of the viral RNA and proteins, but it is also possible to use a helper virus to provide some of the RNA and proteins. Plasmid-based methods using separate plasmids for producing each viral RNA are preferred [35-37], and these methods will also involve the use of plasmids to express all or some (e.g. just the PB 1, PB2, PA and NP proteins) of the viral proteins, with 12 plasmids being used in some methods.

To reduce the number of plasmids needed, a recent approach [38] combines a plurality of RNA polymerase I transcription cassettes (for viral RNA synthesis) on the same plasmid (e.g. sequences encoding 1, 2, 3, 4, 5, 6, 7 or all 8 influenza A vRNA segments), and a plurality of protein-coding regions with RNA polymerase II promoters on another plasmid (e.g. sequences encoding 1, 2, 3, 4, 5, 6, 7 or all 8 influenza A mRNA transcripts). Preferred aspects of the reference 38 method involve: (a) PB1, PB2 and PA mRNA-encoding regions on a single plasmid; and (b) all 8 vRNA-encoding segments on a single plasmid. Including the NA and HA segments on one plasmid and the six other segments on another plasmid can also facilitate matters.

As an alternative to using po1l promoters to encode the viral RNA segments, it is possible to use bacteriophage polymerase promoters [39]. For instance, promoters for the SP6, T3 or T7 polymerases can conveniently be used. Because of the species-specificity of po1l promoters, bacteriophage polymerase promoters can be more convenient for many cell types (e.g. MDCK), although a cell must also be transfected with a plasmid encoding the exogenous polymerase enzyme.

In other techniques it is possible to use dual po1l and po1ll promoters to simultaneously code for the viral RNAs and for expressible mRNAs from a single template [40,41].

Thus an influenza A virus may include one or more RNA segments from a A/PR/8/34 virus (typically 6 segments from A/PR/8/34, with the HA and N segments being from a vaccine strain, i.e. a 6:2 reassortant), particularly when viruses are grown in eggs. It may also include one or more RNA segments from a A/WSN/33 virus, or from any other virus strain useful for generating reassortant viruses for vaccine preparation. Typically, the invention protects against a strain that is capable of human-to-human transmission, and so the strain's genome will usually include at least one RNA segment that originated in a mammalian (e.g. in a human) influenza virus. It may include NS segment that originated in an avian influenza virus.

The viruses used as the source of the antigens can be grown either on eggs (usually SPF eggs) or on cell culture. The current standard method for influenza virus growth uses embryonated hen eggs, with virus being purified from the egg contents (allantoic fluid). More recently, however, viruses have been grown in animal cell culture and, for reasons of speed and patient allergies, this growth method is preferred. If egg-based viral growth is used then one or more amino acids may be introduced into the allantoid fluid of the egg together with the virus [24].

The cell substrate will typically be a cell line of mammalian origin. Suitable mammalian cells of origin include, but are not limited to, hamster, cattle, primate (including humans and monkeys) and dog cells. Various cell types may be used, such as kidney cells, fibroblasts, retinal cells, lung cells, *etc.* Examples of suitable hamster cells are the cell lines having the names BHK21 or HKCC. Suitable monkey cells are e.g. African green monkey cells, such as kidney cells as in the Vero cell line. Suitable dog cells are e.g. kidney cells, as in the MDCK cell line. Thus suitable cell lines include, but are not limited to: MDCK; CHO; 293T; BHK; Vero; MRC-5; PER.C6; WI-38; *etc.* Preferred mammalian cell lines for growing influenza viruses include: MDCK cells [42-45], derived from Madin Darby canine kidney; Vero cells [46-48], derived from African green monkey *(Cercopithecus aethiops)* kidney; or PER.C6 cells [49], derived from human embryonic retinoblasts. These cell lines are widely available e.g. from the American Type Cell Culture (ATCC) collection [50], from the Coriell Cell Repositories [51], or from the European Collection of Cell Cultures (ECACC). For example, the ATCC supplies various different Vero cells under catalog numbers CCL-81, CCL-81.2, CRL-1586 and CRL-1587, and it supplies MDCK cells under catalog number CCL-34. PER.C6 is available from the ECACC under deposit number 96022940. As a less-preferred alternative to mammalian cell lines, virus can be grown on avian cell lines *[e.g.* refs. 52-54], including avian embryonic stem cells [52,55] and cell lines derived from ducks (e.g. duck retina), or from hens. Suitable avian embryonic stem cells, include the EBx cell line derived from chicken embryonic stem cells, EB45, EB14, and EB14-074 [56]. Chicken embryo fibroblasts (CEF), can also be used, *etc.*

The most preferred cell lines for growing influenza viruses are MDCK cell lines. The original MDCK cell line is available from the ATCC as CCL-34, but derivatives of this cell line may also be used. For instance, reference 42 discloses a MDCK cell line that was adapted for growth in suspension culture ('MDCK 33016', deposited as DSM ACC 2219). Similarly, reference 57 discloses a MDCK-derived cell line that grows in suspension in serum-free culture ('B-702', deposited as FERM BP-7449). Reference 58 discloses non-tumorigenic MDCK cells, including 'MDCK-S' (ATCC PTA-6500), 'MDCK-SF101' (ATCC PTA-6501), 'MDCK-SF102' (ATCC PTA-6502) and 'MDCK-SF103' (PTA-6503). Reference 59 discloses MDCK cell lines with high susceptibility to infection, including 'MDCK.5F1' cells (ATCC CRL-12042). Any of these MDCK cell lines can be used.

Where virus has been grown on a mammalian cell line then the composition will advantageously be free from egg proteins (e.g. ovalbumin and ovomucoid) and from chicken DNA, thereby reducing allergenicity.

Where virus has been grown on a cell line then the culture for growth, and also the viral inoculum used to start the culture, will preferably be free from (i.e. will have been tested for and given a negative result for contamination by) herpes simplex virus, respiratory syncytial virus, parainfluenza virus 3, SARS coronavirus, adenovirus, rhinovirus, reoviruses, polyomaviruses, birnaviruses, circoviruses, and/or parvoviruses [60]. Absence of herpes simplex viruses is particularly preferred.

Where virus has been grown on a cell line then the composition preferably contains less than 10ng (preferably less than 1ng, and more preferably less than 100pg) of residual host cell DNA per dose, although trace amounts of host cell DNA may be present. In general, the host cell DNA that it is desirable to exclude from compositions of the invention is DNA that is longer than 100bp. Measurement of residual host cell DNA is now a routine regulatory requirement for biologicals and is within the normal capabilities of the skilled person. The assay used to measure DNA will typically be a validated assay [61,62]. The performance characteristics of a validated assay can be described in mathematical and quantifiable terms, and its possible sources of error will have been identified. The assay will generally have been tested for characteristics such as accuracy, precision, specificity. Once an assay has been calibrated (e.g. against known standard quantities of host cell DNA) and tested then quantitative DNA measurements can be routinely performed. Three principle techniques for DNA quantification can be used: hybridization methods, such as Southern blots or slot blots [63]; immunoassay methods, such as the Threshold^{TM} System [64]; and quantitative PCR [65]. These methods are all familiar to the skilled person, although the precise characteristics of each method may depend on the host cell in question e.g. the choice of probes for hybridization, the choice of primers and/or probes for amplification, *etc.* The Threshold^{TM} system from *Molecular Devices* is a quantitative assay for picogram levels of total DNA, and has been used for monitoring levels of contaminating DNA in biopharmaceuticals [64]. A typical assay involves non-sequence-specific formation of a reaction complex between a biotinylated ssDNA binding protein, a urease-conjugated anti-ssDNA antibody, and DNA. All assay components are included in the complete Total DNA Assay Kit available from the manufacturer. Various commercial manufacturers offer quantitative PCR assays for detecting residual host cell DNA e.g. AppTec^{TM} Laboratory Services, BioReliance^{TM}, Althea Technologies, *etc.* A comparison of a chemiluminescent hybridisation assay and the total DNA Threshold^{TM} system for measuring host cell DNA contamination of a human viral vaccine can be found in reference 66.

Contaminating DNA can be removed during vaccine preparation using standard purification procedures *e.g.* chromatography, *etc.* Removal of residual host cell DNA can be enhanced by nuclease treatment e.g. by using a DNase. A convenient method for reducing host cell DNA contamination is disclosed in references 67 & 68, involving a two-step treatment, first using a DNase (e.g. Benzonase), which may be used during viral growth, and then a cationic detergent (e.g. CTAB) , which may be used during virion disruption. Treatment with an alkylating agent, such as (β-propiolactone, can also be used to remove host cell DNA, and advantageously may also be used to inactivate virions [69].

Vaccines containing <10ng (e.g. <1ng, <100pg) host cell DNA per 15µg of haemagglutinin are preferred, as are vaccines containing <10ng (e.g. <1ng, <100pg) host cell DNA per 0.25ml volume. Vaccines containing <10ng (e.g. <1ng, <100pg) host cell DNA per 50µg of haemagglutinin are more preferred, as are vaccines containing <10ng (e.g. <1ng, <100pg) host cell DNA per 0.5ml volume.

It is preferred that the average length of any residual host cell DNA is less than 500bp e.g. less than 400bp, less than 300bp, less than 200bp, less than 100bp, *etc.*

For growth on a cell line, such as on MDCK cells, virus may be grown on cells in suspension [42,70,71] or in adherent culture. One suitable MDCK cell line for suspension culture is MDCK 33016 (deposited as DSM ACC 2219). As an alternative, microcarrier culture can be used.

Cell lines supporting influenza virus replication are preferably grown in serum-free culture media and/or protein free media. A medium is referred to as a serum-free medium in the context of the present invention in which there are no additives from serum of human or animal origin. Protein-free is understood to mean cultures in which multiplication of the cells occurs with exclusion of proteins, growth factors, other protein additives and non-serum proteins, but can optionally include proteins such as trypsin or other proteases that may be necessary for viral growth. The cells growing in such cultures naturally contain proteins themselves.

Cell lines supporting influenza virus replication are preferably grown below 37°C [72] (e.g. 30-36°C, or at about 30°C, 31 °C, 32°C, 33°C, 34°C, 35°C, 36°C), for example during viral replication.

The method for propagating virus in cultured cells generally includes the steps of inoculating the cultured cells with the strain to be cultured, cultivating the infected cells for a desired time period for virus propagation, such as for example as determined by virus titer or antigen expression (e.g. between 24 and 168 hours after inoculation) and collecting the propagated virus. The cultured cells are inoculated with a virus (measured by PFU or TCID₅₀) to cell ratio of 1:500 to 1:1, preferably 1:100 to 1:5, more preferably 1:50 to 1:10. The virus is added to a suspension of the cells or is applied to a monolayer of the cells, and the virus is absorbed on the cells for at least 60 minutes but usually less than 300 minutes, preferably between 90 and 240 minutes at 25°C to 40°C, preferably 28°C to 37°C. The infected cell culture (e.g. monolayers) may be removed either by freeze-thawing or by enzymatic action to increase the viral content of the harvested culture supernatants. The harvested fluids are then either inactivated or stored frozen. Cultured cells may be infected at a multiplicity of infection ("m.o.i.") of about 0.0001 to 10, preferably 0.002 to 5, more preferably to 0.001 to 2. Still more preferably, the cells are infected at a m.o.i of about 0.01. Infected cells may be harvested 30 to 60 hours post infection. Preferably, the cells are harvested 34 to 48 hours post infection. Still more preferably, the cells are harvested 38 to 40 hours post infection. Proteases (typically trypsin) are generally added during cell culture to allow viral release, and the proteases can be added at any suitable stage during the culture.

Haemagglutinin (HA) is the main immunogen in inactivated influenza vaccines, and vaccine doses are standardised by reference to HA levels, typically as measured by a single radial immunodiffution (SRID) assay. Vaccines typically contain about 15µg of HA per strain, although lower doses are also used e.g. for children, or in pandemic situations. For live vaccines, however, dosing is measured by median tissue culture infectious dose (TCIDₛₒ) rather than by HA content, and a TCIDₛₒ of between 10⁶ and 10⁸ (preferably between 10^{6.5}-10^{7.5}) per strain is typical. Fractional doses such as ¹/₂ (i.e. 7.5µg HA per strain), ¹/₄ and ¹/₈ have been used [73,74], as have higher doses (e.g. 3x or 9x doses [75,76]). Thus vaccines may include between 0.1 and 150µg of HA per influenza strain, preferably between 0.1 and 50µg *e.g.* 0.1-20µg, 0.1-15µg, 0.1-10µg, 0.1-7.5µg, 0.5-5µg, *etc.* Particular doses include e.g. about 45, about 30, about 15, about 10, about 7.5, about 5, about 3.8, about 1.9, about 1.5, *etc.* per strain. These lower doses are most useful when an adjuvant is present in the vaccine, as with the invention. The components of the kits and processes of the invention (e.g. their volumes and concentrations) may be selected to provide these HA doses in the final mixed products.

HA used with the invention may be a natural HA as found in a virus, or may have been modified. For instance, it is known to modify HA to remove determinants (e.g. hyper-basic regions around the cleavage site between HA1 and HA2) that cause a virus to be highly pathogenic in avian species, as these determinants can otherwise prevent a virus from being grown in eggs.

Compositions of the invention may include detergent e.g. a polyoxyethylene sorbitan ester surfactant (known as 'Tweens'), an octoxynol (such as octoxynol-9 (Triton X-100) or t-octylphenoxypolyethoxyethanol), a cetyl trimethyl ammonium bromide ('CTAB'), or sodium deoxycholate, particularly for a split or surface antigen vaccine. The detergent may be present only at trace amounts. Thus the vaccine may included less than 1mg/ml of each of octoxynol-10, α-tocopheryl hydrogen succinate and polysorbate 80. Other residual components in trace amounts could be antibiotics (e.g. neomycin, kanamycin, polymyxin B).

An inactivated but non-whole cell vaccine (e.g. a split virus vaccine or a purified surface antigen vaccine) may include matrix protein, in order to benefit from the additional T cell epitopes that are located within this antigen. Thus a non-whole cell vaccine (particularly a split vaccine) that includes haemagglutinin and neuraminidase may additionally include M1 and/or M2 matrix protein. Where a matrix protein is present, inclusion of detectable levels of M1 matrix protein is preferred. Nucleoprotein may also be present.

### Mixing the two components

Processes of the invention involve mixing two components: (i) an oil-in-water emulsion and (ii) an aqueous preparation of an influenza virus antigen. In a first aspect, substantially equal volumes of (i) and (ii) are used, and component (ii) has a HA concentration of >60µg per influenza virus strain per ml. In a second aspect, an excess volume of (ii) is used. In the first aspect, a standard antigen dose can be achieved with a lower dose volume than with the FLUAD^{TM} product. In the second aspect, the volume of emulsion required for a given amount of antigen can be reduced. In both cases the volume of emulsion required to give a final vaccine is less than used in the existing FLUAD^{TM} product.

Mixing of components can take place during manufacture, to give bulk vaccine, or can take place at the point of use, to give vaccine ready for administration. Where mixing takes place during manufacture then the volumes that are mixed will typically be greater than 1 liter e.g. ≥5 liters, ≥10 liters, ≥20 liters, ≥50 liters, *etc.* Where mixing takes place at the point of use then the volumes that are mixed will typically be smaller than 1 milliliter e.g. ≤0.6ml, ≤0.5ml, ≤0.4ml, ≤0.3ml, ≤0.2ml, *etc.* Where substantially equal volumes of emulsion and antigen solution are used, the ratio of mixed volumes will be substantially 1:1 e.g. between 1.1:1 and 1:1.1, preferably between 1.05:1 and 1:1.05, and more preferably between 1.025:1 and 1:1.025. By using >60µg HA per influenza virus strain per ml then the invention can give a vaccine that delivers the standard HA dose of 15µg/strain, but in a lower administration volume. Whereas the FLUAD^{TM} product requires 0.25ml of emulsion per 15µg/strain, the invention can reduce the amount of emulsion that is required.

Where an excess volume of antigen solution is used, the excess will generally be at least 1.5:1 e.g. ≥2:1, ≥2.5:1, ≥3:1, ≥4:1, ≥5:1, ≥7.5:1, ≥10:1, *etc.* The excess will generally be no more than 25:1 *e.g.* ≤20:1, ≤15:1, ≤10:1, *etc.* The standard 15µg/strain HA dose in a 0.5ml dose of the final product can be achieved by decreasing the HA concentration in the antigen solution to <60µg per strain per ml e.g. if the excess is 3:1 then the HA concentration prior to mixing should be 40µg per strain per ml to give a final concentration of 15µg per strain per 0.5ml dose.

By decreasing the dose by more than the volume excess then a low antigen dose vaccine can be achieved while maintaining dose volume. For example, if a 3:1 excess is used with a 20µg/strain/ml solution then a 0.5ml dose volume will provide a HA dose of 7.5µg per strain.

Antigen concentration and the excess volume ratio can thus be changed to give any desired final antigen concentration and/or dose volume. For example, to give a final antigen dose of 15µg per strain per 0.5ml then, when using an adjuvant:antigen volume ratio of 1:a, the HA concentration in the aqueous antigen preparation should be *30(a+1)*/*a.* A lower HA concentration will provide a final antigen dose of<15µg per strain per 0.5ml.

Where an antigen solution in a process or kit of the invention has a HA concentration of <60µg per influenza virus strain per ml, the level may be, for example, ≤55µg/strain/ml, ≤50µg/strain/ml, ≤45µg/strain/ml, ≤40µg/strain/ml, ≤35µg/strain/ml, ≤30µg/strain/ml, ≤25µg/strain/ml, ≤20µg/strain/ml, ≤15µg/strain/ml, ≤10µg/strain/ml, *etc.* The precise concentration chosen in any given situation may be selected to match any volume reduction that is also being used.

### Pharmaceutical compositions

Compositions of the invention are pharmaceutically acceptable. They may include components in addition to the antigen and adjuvant e.g. they will typically include one or more pharmaceutical carrier(s) and/or excipient(s). A thorough discussion of such components is available in reference 77.

The composition may include preservatives such as thiomersal or 2-phenoxyethanol. It is preferred, however, that the vaccine should be substantially free from (i. e. less than 5µg/ml) mercurial material e.g. thiomersal-free [23,78]. Vaccines containing no mercury are more preferred.

To control tonicity, it is preferred to include a physiological salt, such as a sodium salt. Sodium chloride (NaC1) is preferred, which may be present at between 1 and 20 mg/ml. Other salts that may be present include potassium chloride, potassium dihydrogen phosphate, disodium phosphate dehydrate, magnesium chloride, calcium chloride, *etc.*

Compositions will generally have an osmolality of between 200 mOsm/kg and 400 mOsm/kg, preferably between 240-360 mOsm/kg, and will more preferably fall within the range of 290-310 mOsm/kg. Osmolality has previously been reported not to have an impact on pain caused by vaccination [79], but keeping osmolality in this range is nevertheless preferred.

Compositions may include one or more buffers. Typical buffers include: a phosphate buffer; a Tris buffer; a borate buffer; a succinate buffer; a histidine buffer; or a citrate buffer. Buffers will typically be included in the 5-20mM range.

The pH of a composition will generally be between 5.0 and 8.1, and more typically between 6.0 and 8.0 e.g. between 6.5 and 7.5, or between 7.0 and 7.8. A process of the invention may therefore include a step of adjusting the pH of the bulk vaccine prior to packaging.

The composition is preferably sterile. The composition is preferably non-pyrogenic e.g. containing <1 EU (endotoxin unit, a standard measure) per dose, and preferably <0.1 EU per dose. The composition is preferably gluten free.

The composition may include material for a single immunisation, or may include material for multiple immunisations (i.e. a 'multidose' kit). The inclusion of a preservative is preferred in multidose arrangements. As an alternative (or in addition) to including a preservative in multidose compositions, the compositions may be contained in a container having an aseptic adaptor for removal of material.

Influenza vaccines are typically administered in a dosage volume of about 0.5ml, although a half dose *(i.e.* about 0.25ml) may be administered to children. One advantage of the invention is that it can allow volumes of <0.5ml to be prepared readily.

The antigen and emulsion in a composition will typically be in admixture, although they may initially be presented in the form of a kit of separate components for extemporaneous admixing.

Compositions and kits are preferably stored at between 2°C and 8°C. They should not be frozen. They should ideally be kept out of direct light.

### Kits of the invention

As mentioned above, compositions of the invention may be prepared extemporaneously, at the time of delivery. Thus the invention provides kits including the various components ready for mixing. The kit allows the oil-in-water emulsion and the antigen to be kept separately until the time of use. Any further components may be included in one these two kit components, or may be part of a third kit component.

The components are physically separate from each other within the kit, and this separation can be achieved in various ways. For instance, the components may be in separate containers, such as vials. The contents of two vials can then be mixed e.g. by removing the contents of one vial and adding them to the other vial, or by separately removing the contents of both vials and mixing them in a third container.

In a preferred arrangement, one of the kit components is in a syringe and the other is in a container such as a vial. The syringe can be used (e.g. with a needle) to insert its contents into the second container for mixing, and the mixture can then be withdrawn into the syringe. The mixed contents of the syringe can then be administered to a patient, typically through a new sterile needle. Packing one component in a syringe eliminates the need for using a separate syringe for patient administration.

In another preferred arrangement, the two kit components are held together but separately in the same syringe e.g. a dual-chamber syringe, such as those disclosed in references 80-87 *etc.* When the syringe is actuated (e.g. during administration to a patient) then the contents of the two chambers are mixed. This arrangement avoids the need for a separate mixing step at the time of use.

Where the invention uses an excess volume of antigen solution then the volumes of vials, syringe compartments, etc., will be changed accordingly.

The contents of the various kit components will generally all be in aqueous form. In some arrangements, a component (typically the antigen component rather than the emulsion component) is in dry form (e.g. in a lyophilised form), with the other component being in aqueous form. The two components can be mixed in order to reactivate the dry component and give an aqueous composition for administration to a patient. A lyophilised component will typically be located within a vial rather than a syringe. Dried components may include stabilizers such as lactose, sucrose or mannitol, as well as mixtures thereof e.g. lactose/sucrose mixtures, sucrose/mannitol mixtures, *etc.* One possible arrangement uses an aqueous emulsion component in a pre-filled syringe and a lyophilised antigen component in a vial.

### Packaging of compositions or kit components

Suitable containers for compositions of the invention (or kit components) include vials, syringes (e.g. disposable syringes), nasal sprays, *etc..* These containers should be sterile.

Where a composition/component is located in a vial, the vial is preferably made of a glass or plastic material. The vial is preferably sterilized before the composition is added to it. To avoid problems with latex-sensitive patients, vials are preferably sealed with a latex-free stopper, and the absence of latex in all packaging material is preferred. The vial may include a single dose of vaccine, or it may include more than one dose (a 'multidose' vial) e.g. 10 doses. Preferred vials are made of colorless glass.

A vial can have a cap (e.g. a Luer lock) adapted such that a pre-filled syringe can be inserted into the cap, the contents of the syringe can be expelled into the vial (e.g. to reconstitute lyophilised material therein), and the contents of the vial can be removed back into the syringe. After removal of the syringe from the vial, a needle can then be attached and the composition can be administered to a patient. The cap is preferably located inside a seal or cover, such that the seal or cover has to be removed before the cap can be accessed. A vial may have a cap that permits aseptic removal of its contents, particularly for multidose vials.

Where a component is packaged into a syringe, the syringe may have a needle attached to it. If a needle is not attached, a separate needle may be supplied with the syringe for assembly and use. Such a needle may be sheathed. Safety needles are preferred. 1-inch 23-gauge, 1-inch 25-gauge and 5/8-inch 25-gauge needles are typical. Syringes may be provided with peel-off labels on which the lot number, influenza season and expiration date of the contents may be printed, to facilitate record keeping. The plunger in the syringe preferably has a stopper to prevent the plunger from being accidentally removed during aspiration. The syringes may have a latex rubber cap and/or plunger. Disposable syringes contain a single dose of vaccine. The syringe will generally have a tip cap to seal the tip prior to attachment of a needle, and the tip cap is preferably made of a butyl rubber. If the syringe and needle are packaged separately then the needle is preferably fitted with a butyl rubber shield. Preferred syringes are those marketed under the trade name "Tip-Lok"^{TM}.

Containers may be marked to show a half-dose volume e.g. to facilitate delivery to children. For instance, a syringe containing a 0.5ml dose may have a mark showing a 0.25ml volume.

Where a glass container (e.g. a syringe or a vial) is used, then it is preferred to use a container made from a borosilicate glass rather than from a soda lime glass.

A kit or composition may be packaged (e.g. in the same box) with a leaflet including details of the vaccine e.g. instructions for administration, details of the antigens within the vaccine, *etc.* The instructions may also contain warnings e.g. to keep a solution of adrenaline readily available in case of anaphylactic reaction following vaccination, etc.

### Methods of treatment, and administration of the vaccine

Compositions of the invention are suitable for administration to human patients, and the invention provides a method of raising an immune response in a patient, comprising the step of administering a composition of the invention to the patient.

The invention also provides a kit or composition of the invention for use as a medicament.

The invention also provides the use of substantially equal volumes of: (i) an aqueous preparation of an influenza virus antigen, with a hemagglutinin concentration of more than 60µg per influenza virus strain per ml; and (ii) an oil-in-water emulsion adjuvant, in the manufacture of a medicament for raising an immune response in a patient.

The invention also provides the use of substantially equal volumes of: (i) an aqueous preparation of an influenza virus antigen, with a hemagglutinin concentration of less than 60µg per influenza virus strain per ml; and (ii) an oil-in-water emulsion adjuvant, in the manufacture of a medicament for raising an immune response in a patient.

The invention also provides the use of: (i) an aqueous preparation of an influenza virus antigen, having a first volume; and (ii) an oil-in-water emulsion adjuvant, having a second volume, in the manufacture of a medicament for raising an immune response in a patient, the second volume is less than the first volume.

The immune response raised by these methods and uses will generally include an antibody response, preferably a protective antibody response. Methods for assessing antibody responses, neutralising capability and protection after influenza virus vaccination are well known in the art. Human studies have shown that antibody titers against hemagglutinin of human influenza virus are correlated with protection (a serum sample hemagglutination-inhibition titer of about 30-40 gives around 50% protection from infection by a homologous virus) [88]. Antibody responses are typically measured by hemagglutination inhibition, by microneutralisation, by single radial immunodiffusion (SRID), and/or by single radial hemolysis (SRH). These assay techniques are well known in the art.

Compositions of the invention can be administered in various ways. The most preferred immunisation route is by intramuscular injection (e.g. into the arm or leg), but other available routes include subcutaneous injection, intranasal [89-91], oral [92], intradermal [93,94], transcutaneous, transdermal [95], *etc.*

Vaccines prepared according to the invention may be used to treat both children and adults. Influenza vaccines are currently recommended for use in pediatric and adult immunisation, from the age of 6 months. Thus the patient may be less than 1 year old, 1-5 years old, 5-15 years old, 15-55 years old, or at least 55 years old. Preferred patients for receiving the vaccines are the elderly (e.g. ≥50 years old, ≥60 years old, preferably ≥65 years), the young (e.g. ≥5 years old), hospitalised patients, healthcare workers, armed service and military personnel, pregnant women, the chronically ill, immunodeficient patients, patients who have taken an antiviral compound (e.g. an oseltamivir or zanamivir compound, such as oseltamivir phosphate; see below) in the 7 days prior to receiving the vaccine, and people travelling abroad. The vaccines are not suitable solely for these groups, however, and may be used more generally in a population. For pandemic strains, administration to all age groups is preferred.

Treatment can be by a single dose schedule or a multiple dose schedule. Multiple doses may be used in a primary immunisation schedule and/or in a booster immunisation schedule. In a multiple dose schedule the various doses may be given by the same or different routes e.g. a parenteral prime and mucosal boost, a mucosal prime and parenteral boost, etc. Administration of more than one dose (typically two doses) is particularly useful in immunologically naïve patients e.g. for people who have never received an influenza vaccine before, or for vaccinating against a new HA subtype (as in a pandemic outbreak). Multiple doses will typically be administered at least 1 week apart (e.g. about 2 weeks, about 3 weeks, about 4 weeks, about 6 weeks, about 8 weeks, about 10 weeks, about 12 weeks, about 16 weeks, *etc.).*

Preferred compositions of the invention satisfy 1, 2 or 3 of the CPMP criteria for efficacy. In adults (18-60 years), these criteria are: (1) ≥70% seroprotection; (2) ≥40% seroconversion; and/or (3) a GMT increase of ≥2.5-fold. In elderly (>60 years), these criteria are: (1) ≥60% seroprotection; (2) ≥30% seroconversion; and/or (3) a GMT increase of ≥2-fold. These criteria are based on open label studies with at least 50 patients.

Vaccines of the invention may be administered to patients at substantially the same time as *(e.g.* during the same medical consultation or visit to a healthcare professional) other vaccines e.g. at substantially the same time as a measles vaccine, a mumps vaccine, a rubella vaccine, a MMR vaccine, a varicella vaccine, a MMRV vaccine, a diphtheria vaccine, a tetanus vaccine, a pertussis vaccine, a DTP vaccine, a conjugated *H. influenzae* type b vaccine, an inactivated poliovirus vaccine, a hepatitis B virus vaccine, a meningococcal conjugate vaccine (such as a tetravalent A-C-W135-Y vaccine), a respiratory syncytial virus vaccine, a pneumococcal conjugate vaccine, *etc.* Administration at substantially the same time as a pneumococcal vaccine and/or a meningococcal vaccine is particularly useful in elderly patients.

Similarly, vaccines of the invention may be administered to patients at substantially the same time as (e.g. during the same medical consultation or visit to a healthcare professional) an antiviral compound, and in particular an antiviral compound active against influenza virus (e.g. oseltamivir and/or zanamivir). These antivirals include neuraminidase inhibitors, such as a (3R,4R,5S)-4-acetylamino-5-amino-3(1-ethylpropoxy)-1-cyclohexene-1-carboxylic acid or 5-(acetylamino)-4-[(aminoiminomethyl)-amino]-2,6-anhydro-3,4,5-trideoxy-D-glycero-D-galactonon-2-enonic acid, including esters thereof *(e.g.* the ethyl esters) and salts thereof *(e.g.* the phosphate salts). A preferred antiviral is (3R,4R,5S)-4-acetylamino-5-amino-3(1-ethylpropoxy)-1-cyclohexene-l-carboxylic acid, ethyl ester, phosphate (1:1), also known as oseltamivir phosphate (TAMIFLU^{TM}).

### Cytokine-inducing agents

Compositions of the invention may include a cytokine-inducing agent, and it has been found that the combination of this agent with an oil-in-water emulsion gives a surprisingly effective immunogenic composition, with a synergistic effect on T cell responses. T cell responses are reported to be better able than antibody responses to resist influenza virus antigenic drift. Moreover, T cell effector mechanisms may be an important determinant of vaccine-induced protection against serious illness in elderly patients [96], and it may be possible to diminish age-related susceptibility to influenza by inducing a more potent interferon-γ response [97].

The cytokine-inducing agents for inclusion in compositions of the invention are able, when administered to a patient, to elicit the immune system to release cytokines, including interferons and interleukins. Preferred agents can elicit the release of one or more of: interferon-γ; interleukin-1; interleukin-2; interleukin-12; TNF-α; TNF-β; and GM-CSF. Preferred agents elicit the release of cytokines associated with a Th1-type immune response e.g. interferon-γ, TNF-α, interleukin-2. Stimulation of both interferon-γ and interleukin-2 is preferred.

As a result of receiving these compositions, therefore, a patient will have T cells that, when stimulated with an influenza antigen, will release the desired cytokine(s) in an antigen-specific manner. For example, T cells purified form their blood will release γ-interferon when exposed *in vitro* to influenza virus haemagglutinin. Methods for measuring such responses in peripheral blood mononuclear cells (PBMC) are known in the art, and include ELISA, ELISPOT, flow-cytometry and real-time PCR. For example, reference 98 reports a study in which antigen-specific T cell-mediated immune responses against tetanus toxoid, specifically γ-interferon responses, were monitored, and found that ELISPOT was the most sensitive method to discriminate antigen-specific TT-induced responses from spontaneous responses, but that intracytoplasmic cytokine detection by flow cytometry was the most efficient method to detect re-stimulating effects.

Suitable cytokine-inducing agents include, but are not limited to:
- An immunostimulatory oligonucleotide, such as one containing a CpG motif (a dinucleotide sequence containing an unmethylated cytosine linked by a phosphate bond to a guanosine), or a double-stranded RNA, or an oligonucleotide containing a palindromic sequence, or an oligonucleotide containing a poly(dG) sequence.
- 3-O-deacylated monophosphoryl lipid A ('3dMPL', also known as `MPL^{TM}') [99-102].
- An imidazoquinoline compound, such as Imiquimod ("R837") [103,104], Resiquimod ("R-848") [105], and their analogs; and salts thereof (e.g. the hydrochloride salts). Further details about immunostimulatory imidazoquinolines can be found in references 106 to 110.
- A thiosemicarbazone compound, such as those disclosed in reference 111. Methods of formulating, manufacturing, and screening for active compounds are also described in reference 111. The thiosemicarbazones are particularly effective in the stimulation of human peripheral blood mononuclear cells for the production of cytokines, such as TNF-α.
- tryptanthrin compound, such as those disclosed in reference 112. Methods of formulating, manufacturing, and screening for active compounds are also described in reference 112. The thiosemicarbazones are particularly effective in the stimulation of human peripheral blood mononuclear cells for the production of cytokines, such as TNF-α.
- A nucleoside analog, such as: (a) Isatorabine (ANA-245; 7-thia-8-oxoguanosine): 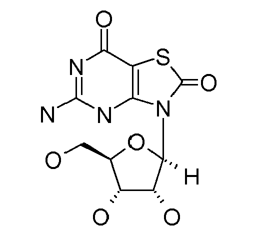 and prodrugs thereof; (b)ANA975; (c) ANA-025-1; (d) ANA380; (e) the compounds disclosed in references 113 to 115; (f) a compound having the formula: 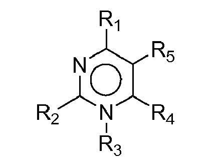 wherein:
   R₁ and R₂ are each independently H, halo, -NRₐR_{b}, -OH, C₁-₆ alkoxy, substituted C₁-₆ alkoxy, heterocyclyl, substituted heterocyclyl, C₆-₁₀ aryl, substituted C₆-₁₀ aryl, C₁-₆ alkyl, or substituted C₁₋₆ alkyl;
   R₃ is absent, H, C₁₋₆ alkyl, substituted C₁₋₆ alkyl, C₆₋₁₀ aryl, substituted C₆₋₁₀ aryl, heterocyclyl, or substituted heterocyclyl;
   R₄ and R₅ are each independently H, halo, heterocyclyl, substituted heterocyclyl, - C(O)-R_{d}, C₁₋₆ alkyl, substituted C₁₋₆ alkyl, or bound together to form a 5 membered ring as in R₄₋₅: 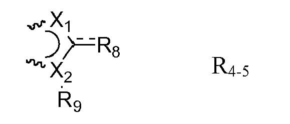 the binding being achieved at the bonds indicated by a 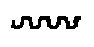
   X₁ and X₂ are each independently N, C, O, or S;
   R₈ is H, halo, -OH, C₁₋₆ alkyl, C₂₋₆ alkenyl, C₂₋₆ alkynyl, -OH, -NRₐR_{b}, -(CH₂)n-O-R_{c}, -O-(C₁₋₆ alkyl), -S(O)pRₑ, or -C(O)-R_{d};
   R₉ is H, C₁₋₆ alkyl, substituted C₁₋₆ alkyl, heterocyclyl, substituted heterocyclyl or R₉ₐ, wherein R₉ₐ is: 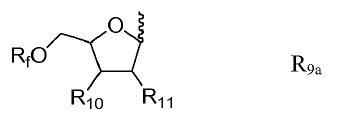 the binding being achieved at the bond indicated by a 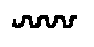
   R₁₀ and R₁₁ are each independently H, halo, C₁₋₆ alkoxy, substituted C₁₋₆ alkoxy, - NRₐR_{b}, or -OH;
   each Rₐ and R_{b} is independently H, C₁₋₆ alkyl, substituted C₁₋₆ alkyl, -C(O)R_{d}, C₆₋₁₀ aryl;
   each R_{c} is independently H, phosphate, diphosphate, triphosphate, C₁₋₆ alkyl, or substituted Cₗ-₆ alkyl;
   each R_{d} is independently H, halo, C₁₋₆ alkyl, substituted C₁₋₆ alkyl, C₁₋₆ alkoxy, substituted C₁₋₆ alkoxy, -NH₂, -NH(C₁₋₆ alkyl), -NH(substituted C₁₋₆ alkyl), -N(C₁₋₆ alkyl)₂, -N(substituted C₁₋₆ alkyl)₂, C₆₋₁₀ aryl, or heterocyclyl;
   each Rₑ is independently H, C₁₋₆ alkyl, substituted C₁₋₆ alkyl, C₆₋₁₀ aryl, substituted C₆₋₁₀ aryl, heterocyclyl, or substituted heterocyclyl;
   each R_{f} is independently H, C₁₋₆ alkyl, substituted C₁₋₆ alkyl, -C(O)R_{d}, phosphate, diphosphate, or triphosphate;
   each n is independently 0, 1, 2, or 3;
   each p is independently 0, 1, or 2; or
   or (g) a pharmaceutically acceptable salt of any of (a) to (f), a tautomer of any of (a) to (f), or a pharmaceutically acceptable salt of the tautomer.
- Loxoribine (7-allyl-8-oxoguanosine) [116].
- Compounds disclosed in reference 117, including: Acylpiperazine compounds, Indoledione compounds, Tetrahydraisoquinoline (THIQ) compounds, Benzocyclodione compounds, Aminoazavinyl compounds, Aminobenzimidazole quinolinone (ABIQ) compounds [118,119], Hydrapthalamide compounds, Benzophenone compounds, Isoxazole compounds, Sterol compounds, Quinazilinone compounds, Pyrrole compounds [120], Anthraquinone compounds, Quinoxaline compounds, Triazine compounds, Pyrazalopyrimidine compounds, and Benzazole compounds [121].
- Compounds disclosed in reference 122.
- A compound of formula I, II or III, or a salt thereof: 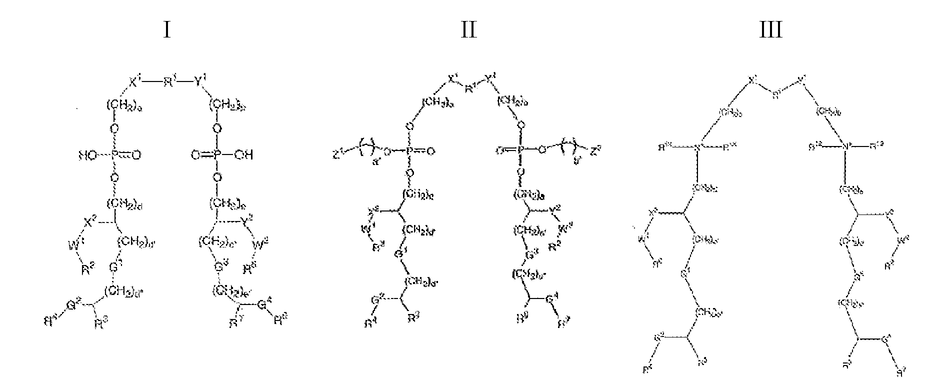 as defined in reference 123, such as 'ER 803058', 'ER 803732', 'ER 804053', ER 804058', 'ER 804059', `ER 804442', `ER 804680', `ER 804764', `ER 804057' (structure below): 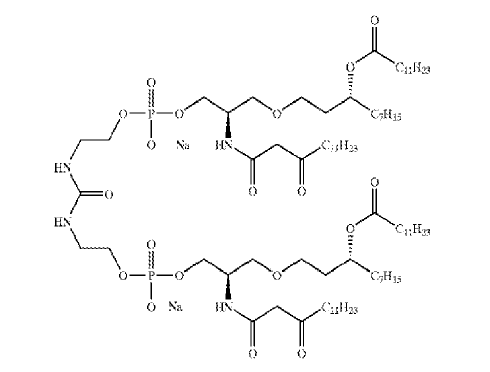 or ER-803022 (structure shown below): 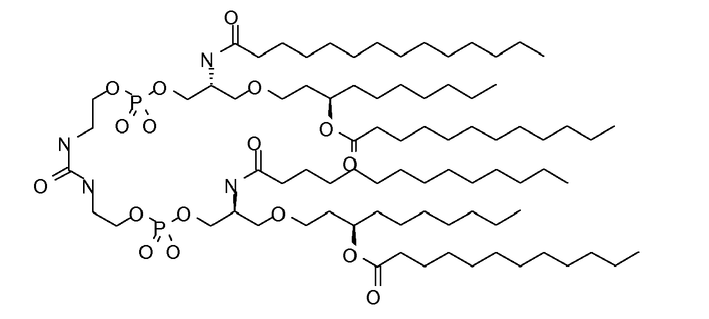
- An aminoalkyl glucosaminide phosphate derivative, such as RC-529 [124,125]. The ability of RC-529 to stimulate cytokine responses in CD4⁺ T cells is reported in reference 126.
- A phosphazene, such as poly[di(carboxylatophenoxy)phosphazene] ("PCPP") as described, for example, in references 127 and 128.
- Compounds containing lipids linked to a phosphate-containing acyclic backbone, such as the TLR4 antagonist E5564 [129,130]: 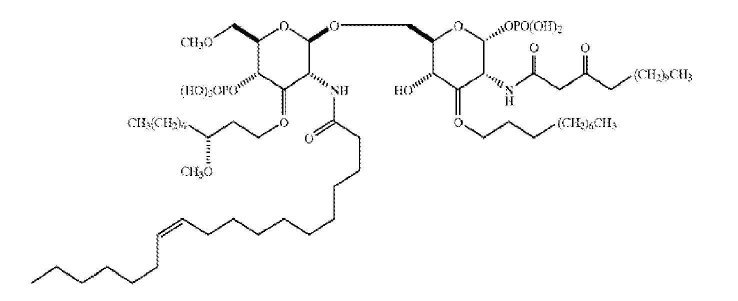
- Small molecule immunopotentiators (SMIPs) such as:
   N2-methyl-1-(2-methylpropyl)-1H-imidazo[4,5-c]quinoline-2,4-diamine;
   N2,N2-dimethyl-1-(2-methylpropyl)-1H-imidazo[4,5-c]quinoline-2,4-diamine;
   N2-ethyl-N2-methyl-1-(2-methylpropyl)-1H-imidazo[4,5-c]quinoline-2,4-diamine;
   N2-methyl-1-(2-methylpropyl)-N2-propyl-1H-imidazo[4,5-c]quinoline-2,4-diamine;
   1-(2-methylpropyl)-N2-propyl-1H-imidazo[4,5-c]quinoline-2,4-diamine;
   N2-butyl-1-(2-methylpropyl)-1H-imidazo[4,5-c]quinoline-2,4-diamine;
   N2-butyl-N2-methyl-1-(2-methylpropyl)-1H-imidazo[4,5-c]quinoline-2,4-diamine;
   N2-methyl-1-(2-methylpropyl)-N2-pentyl-1H-imidazo[4,5-c]quinoline-2,4-diamine;
   N2-methyl-1-(2-methylpropyl)-N2-prop-2-enyl-1H-imidazo[4,5-c]quinoline-2,4-diamine;
   1-(2-methylpropyl)-2-[(phenylmethyl)thio]-1H-imidazo[4,5-c]quinolin-4-amine;
   1-(2-methylpropyl)-2-(propylthio)-1H-imidazo[4,5-c]quinolin-4-amine ;
   2-[[4-amino-1-(2-methylpropyl)-1H-imidazo[4,5-c]quinolin-2-y1](methyl)amino]ethanol;
   2-[[4-amino-1-(2-methylpropyl)-1H-imidazo[4,5-c]quinolin-2-yl](methyl)amino]ethyl acetate;
   4-amino-1-(2-methylpropyl)-1,3-dihydro-2H-imidazo[4,5-c]quinolin-2-one;
   N2-butyl-1-(2-methylpropyl)-N4,N4-bis(phenylmethyl)-1H-imidazo[4,5-c]quinoline-2,4-diamine;
   N2-butyl-N2-methyl-1-(2-methylpropyl)-N4,N4-bis(phenylmethyl)-1H-imidazo[4,5-c]quinoline-2,4-diamine;
   N2-methyl-1-(2-methylpropyl)-N4,N4-bis(phenylmethyl)-1H-imidazo[4,5-c]quinoline-2,4-diamine;
   N2,N2-dimethyl-1-(2-methylpropyl)-N4,N4-bis(phenylmethyl)-1H-imidazo[4,5-c]quinoline-2,4-diamine;
   1- {4-amino-2-[ methyl(propyl)amino ]-1H -imidazo[4,5-c]quinolin-1-y1} -2-methylpropan-2-ol;
   1-[4-amino-2-(propylamino)-1H-imidazo[4,5-c]quinolin-1-y1]-2-methylpropan-2-ol;
   N4,N4-dibenzyl-1-(2-methoxy-2-methylpropyl)-N2-propyl-1H-imidazo[4,5-c]quinoline-2,4-diamine.

The cytokine-inducing agents for use in the present invention may be modulators and/or agonists of Toll-Like Receptors (TLR). For example, they may be agonists of one or more of the human TLR1, TLR2, TLR3, TLR4, TLR7, TLR8, and/or TLR9 proteins. Preferred agents are agonists of TLR7 (e.g. imidazoquinolines) and/or TLR9 *(e.g.* CpG oligonucleotides). These agents are useful for activating innate immunity pathways.

A cytokine-inducing agent can be added to the composition at various stages during its production. For example, it may be within an antigen composition, and this mixture can then be added to an oil-in-water emulsion. As an alternative, it may be within an oil-in-water emulsion, in which case the agent can either be added to the emulsion components before emulsification, or it can be added to the emulsion after emulsification. Similarly, the agent may be coacervated within the emulsion droplets. The location and distribution of the cytokine-inducing agent within the final composition will depend on its hydrophilic/lipophilic properties e.g. the agent can be located in the aqueous phase, in the oil phase, and/or at the oil-water interface.

The cytokine-inducing agent can be conjugated to a separate agent, such as an antigen (e.g. CRM197). A general review of conjugation techniques for small molecules is provided in ref. 131. As an alternative, the adjuvants may be non-covalently associated with additional agents, such as by way of hydrophobic or ionic interactions.

Two preferred cytokine-inducing agents are (a) immunostimulatory oligonucleotides and (b) 3dMPL.

### Immunostimulatory oligonucleotides

Immunostimulatory oligonucleotides can include nucleotide modifications/analogs such as phosphorothioate modifications and can be double-stranded or (except for dsRNA) single-stranded. References 132, 133 and 134 disclose possible analog substitutions e.g. replacement of guanosine with 2'-deoxy-7-deazaguanosine. The adjuvant effect of CpG oligonucleotides is further discussed in refs. 135-140. The CpG sequence may be directed to TLR9, such as the motif GTCGTT or TTCGTT [141]. The CpG sequence may be specific for inducing a Th1 immune response, such as a CpG-A ODN (oligodeoxynucleotide), or it may be more specific for inducing a B cell response, such a CpG-B ODN. CpG-A and CpG-B ODNs are discussed in refs. 142-144. Preferably, the CpG is a CpG-A ODN. Preferably, the CpG oligonucleotide is constructed so that the 5' end is accessible for receptor recognition. Optionally, two CpG oligonucleotide sequences may be attached at their 3' ends to form "immunomers". See, for example, references 141 & 145-147. A useful CpG adjuvant is CpG7909, also known as ProMune^{TM} (Coley Pharmaceutical Group, Inc.).

As an alternative, or in addition, to using CpG sequences, TpG sequences can be used [148]. These oligonucleotides may be free from unmethylated CpG motifs.

The immunostimulatory oligonucleotide may be pyrimidine-rich. For example, it may comprise more than one consecutive thymidine nucleotide (e.g. TTTT, as disclosed in ref. 148), and/or it may have a nucleotide composition with >25% thymidine *(e.g.* >35%, >40%, >50%, >60%, >80%, *etc.).* For example, it may comprise more than one consecutive cytosine nucleotide (e.g. CCCC, as disclosed in ref. 148), and/or it may have a nucleotide composition with >25% cytosine *(e.g.* >35%, >40%, >50%, >60%, >80%, *etc.).* These oligonucleotides may be free from unmethylated CpG motifs.

Immunostimulatory oligonucleotides will typically comprise at least 20 nucleotides. They may comprise fewer than 100 nucleotides.

### 3dMPL

3dMPL (also known as 3 de-O-acylated monophosphoryl lipid A or 3-O-desacyl-4'-monophosphoryl lipid A) is an adjuvant in which position 3 of the reducing end glucosamine in monophosphoryl lipid A has been de-acylated. 3dMPL has been prepared from a heptoseless mutant of *Salmonella minnesota,* and is chemically similar to lipid A but lacks an acid-labile phosphoryl group and a base-labile acyl group. It activates cells of the monocyte/macrophage lineage and stimulates release of several cytokines, including IL-1, IL-12, TNF-α and GM-CSF (see also ref. 126). Preparation of 3dMPL was originally described in reference 149.

3dMPL can take the form of a mixture of related molecules, varying by their acylation (e.g. having 3, 4, 5 or 6 acyl chains, which may be of different lengths). The two glucosamine (also known as 2-deoxy-2-amino-glucose) monosaccharides are N-acylated at their 2-position carbons (i.e. at positions 2 and 2'), and there is also O-acylation at the 3' position. The group attached to carbon 2 has formula -NH-CO-CH₂-CR¹R^{1'}. The group attached to carbon 2' has formula -NH-CO-CH₂-CR²R^{2'}. The group attached to carbon 3' has formula -O-CO-CH₂-CR ³R^{3'} . A representative structure is:

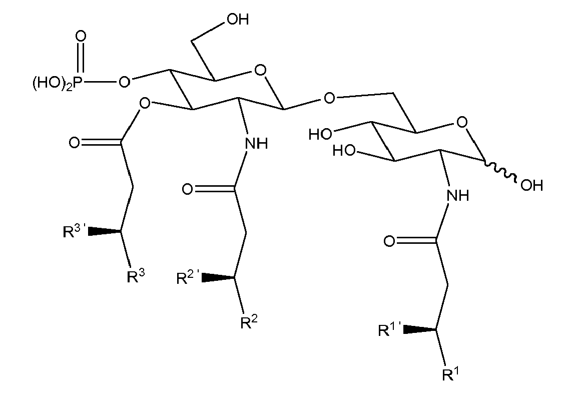

Groups R¹, R² and R³ are each independently ―(CH₂)n―CH₃. The value of n is preferably between 8 and 16, more preferably between 9 and 12, and is most preferably 10.

Groups R^{1'}, R^{2'} and R^{3'} can each independently be: (a) ―H; (b) ―OH; or (c) ―O-CO-R⁴,where R⁴ is either ―H or ―(CH₂)ₘ―CH₃, wherein the value of m is preferably between 8 and 16, and is more preferably 10, 12 or 14. At the 2 position, m is preferably 14. At the 2' position, m is preferably 10. At the 3' position, m is preferably 12. Groups R^{1'}, R^{2'} and R^{3'} are thus preferably -O-acyl groups from dodecanoic acid, tetradecanoic acid or hexadecanoic acid.

When all of R^{1'}, R^{2'} and R^{3'} are ―H then the 3dMPL has only 3 acyl chains (one on each of positions 2, 2' and 3'). When only two of R^{1'}, R^{2'} and R^{3'} are ―H then the 3dMPL can have 4 acyl chains. When only one of R^{1'}, R^{2'} and R^{3'} is ―H then the 3dMPL can have 5 acyl chains. When none of R^{1'}, R^{2'} and R^{3'} is ―H then the 3dMPL can have 6 acyl chains. The 3dMPL adjuvant used according to the invention can be a mixture of these forms, with from 3 to 6 acyl chains, but it is preferred to include 3dMPL with 6 acyl chains in the mixture, and in particular to ensure that the hexaacyl chain form makes up at least 10% by weight of the total 3dMPL e.g. >20%, ≥30%, ≥40%, ≥50% or more. 3dMPL with 6 acyl chains has been found to be the most adjuvant-active form.

Thus the most preferred form of 3dMPL for inclusion in compositions of the invention is:

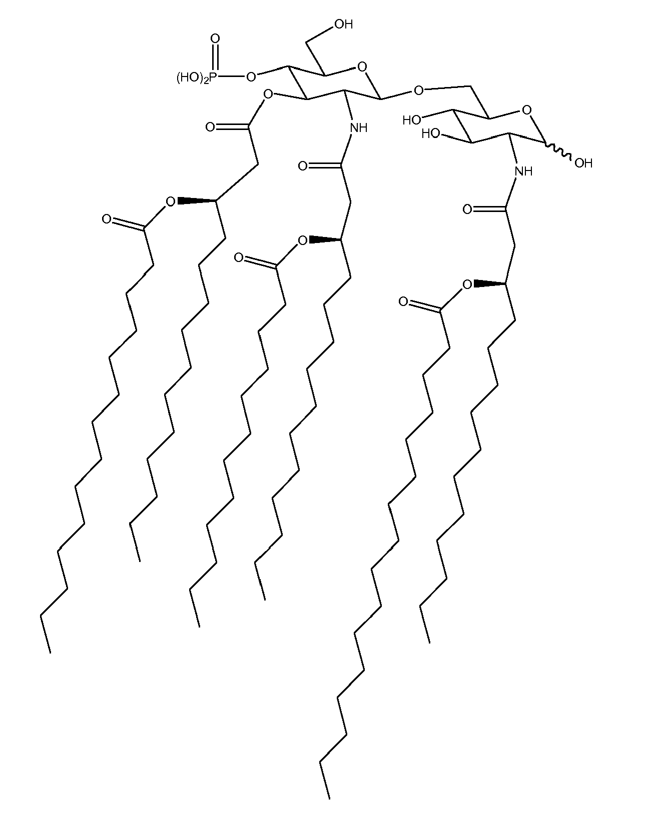

Where 3dMPL is used in the form of a mixture then references to amounts or concentrations of 3dMPL in compositions of the invention refer to the combined 3dMPL species in the mixture.

In aqueous conditions, 3dMPL can form micellar aggregates or particles with different sizes e.g. with a diameter <150nm or >500nm. Either or both of these can be used with the invention, and the better particles can be selected by routine assay. Smaller particles (e.g. small enough to give a clear aqueous suspension of 3dMPL) are preferred for use according to the invention because of their superior activity [150]. Preferred particles have a mean diameter less than 220nm, more preferably less than 200nm or less than 150nm or less than 120nm, and can even have a mean diameter less than 100nm. In most cases, however, the mean diameter will not be lower than 50nm. These particles are small enough to be suitable for filter sterilization. Particle diameter can be assessed by the routine technique of dynamic light scattering, which reveals a mean particle diameter. Where a particle is said to have a diameter ofx nm, there will generally be a distribution of particles about this mean, but at least 50% by number (e.g. ≥60%, ≥70%, ≥80%, ≥90%, or more) of the particles will have a diameter within the range x±25%.

Substantially all of the 3dMPL is preferably located in the aqueous phase of the emulsion.

A typical amount of 3dMPL in a vaccine is 10-100µg/dose e.g. about 25µg or about 50µg.

The 3dMPL can be used on its own, or in combination with one or more further compounds. For example, it is known to use 3dMPL in combination with the QS21 saponin [151] (including in an emulsion [152]), with aluminum phosphate [153], or with aluminum hydroxide [154].

### General

The term "comprising" encompasses "including" as well as "consisting" e.g. a composition "comprising" X may consist exclusively of X or may include something additional e.g. X + Y.

The word "substantially" does not exclude "completely" e.g. a composition which is "substantially free" from Y may be completely free from Y. Where necessary, the word "substantially" may be omitted from the definition of the invention.

The term "about" in relation to a numerical value x means, for example, x±10%.

Unless specifically stated, a process comprising a step of mixing two or more components does not require any specific order of mixing. Thus components can be mixed in any order. Where there are three components then two components can be combined with each other, and then the combination may be combined with the third component, etc.

Where animal (and particularly bovine) materials are used in the culture of cells, they should be obtained from sources that are free from transmissible spongiform encaphalopathies (TSEs), and in particular free from bovine spongiform encephalopathy (BSE). Overall, it is preferred to culture cells in the total absence of animal-derived materials.

Where a compound is administered to the body as part of a composition then that compound may alternatively be replaced by a suitable prodrug.

Where a cell substrate is used for reassortment or reverse genetics procedures, it is preferably one that has been approved for use in human vaccine production e.g. as in Ph Eur general chapter 5.2.3.

The invention further provides the following non-limiting embodiments:
1. A process for making an adjuvanted influenza vaccine, comprising a step of mixing substantially equal volumes of (i) an oil-in-water emulsion and (ii) an aqueous preparation of an influenza virus antigen, wherein the concentration of hemagglutinin in component (ii) is more than 60µg per influenza virus strain per ml.
2. The process of embodiment 1, where the volume of a unit dose of the vaccine is less than 0.5ml.
3. A process for making an adjuvanted influenza vaccine, comprising a step of mixing substantially equal volumes of (i) an oil-in-water emulsion and (ii) an aqueous preparation of an influenza virus antigen, wherein the volume of a unit dose of the vaccine is less than 0.5ml.
4. A process for making an adjuvanted influenza vaccine, comprising the steps of: (a) mixing substantially equal volumes of (i) an oil-in-water emulsion and (ii) an aqueous preparation of an influenza virus antigen, to give a bulk vaccine; and (b) removing at least one unit dose from the bulk vaccine, where the volume of the unit dose is less than 0.5ml.
5. The process of embodiment 3 or embodiment 4, where the concentration of hemagglutinin in component (ii) is about 60µg per influenza virus strain per ml.
6. A process for making an adjuvanted influenza vaccine, comprising a step of mixing substantially equal volumes of (i) an oil-in-water emulsion and (ii) an aqueous preparation of an influenza virus antigen, wherein the concentration of hemagglutinin in the vaccine is less than 30µg per influenza virus strain per ml.
7. A process for making an adjuvanted influenza vaccine, comprising the step of mixing a first volume of an oil-in-water emulsion with a second volume of an aqueous preparation of an influenza virus antigen, wherein the second volume is greater than the first volume.
8. The process of embodiment 7, wherein the concentration of hemagglutinin in the second volume is about 60µg per influenza virus strain per ml.
9. The process of embodiment 7, wherein the concentration of hemagglutinin in the second volume is less than 60µg per influenza virus strain per ml.
10. Compositions obtainable by the process of any preceding embodiment.
11. A vaccine composition comprising an oil-in-water emulsion and influenza virus antigen, wherein the vaccine has a volume of less than 0.5ml.
12. A vaccine composition comprising influenza virus haemagglutinin and squalene, wherein the weight ratio of squalene to haemagglutinin is between 50 and 2000 and wherein the vaccine has a volume of less than 0.5ml.
13. The vaccine of embodiment 12, with a HA concentration of about 30µg per strain per ml.
14. The vaccine of embodiment 12, with a HA concentration of >30µg per strain per ml.
15. The vaccine of embodiment 12, with a HA concentration of <30µg per strain per ml.
16. A composition comprising an oil in water emulsion and hemagglutinin from n influenza virus strains, wherein the weight ratio of oil to hemagglutinin is less than 640/n.
17. The composition of embodiment 16, wherein the value of n is 1, 2, 3 or 4.
18. The process or composition of any preceding embodiment, wherein the oil-in-water emulsion includes squalene.
19. The process or composition of any preceding embodiment, wherein the oil-in-water emulsion includes polysorbate 80.
20. The process or composition of any preceding embodiment, wherein the influenza virus antigen is inactivated virus.
21. The process or composition of embodiment 20, wherein the influenza virus antigen comprises whole virus, split virus, or purified surface antigens.
22. The process or composition of any preceding embodiment, wherein the influenza virus antigen is from a H1, H2, H3, H5, H7 or H9 influenza A virus subtype.
23. The process or composition of any preceding embodiment, wherein the influenza virus antigen is prepared from an influenza virus grown on eggs.
24. The process or composition of any preceding embodiment, wherein the influenza virus antigen is prepared from an influenza virus grown on cell culture.
25. The process or composition of embodiment 24, wherein the cell culture is a MDCK cell culture.
26. The process or composition of embodiment 24, wherein the composition is free from ovalbumin, ovomucoid and chicken DNA.
27. A kit for preparing the composition of any one of embodiments 10 to 26.

### BRIEF DESCRIPTION OF THE DRAWINGS

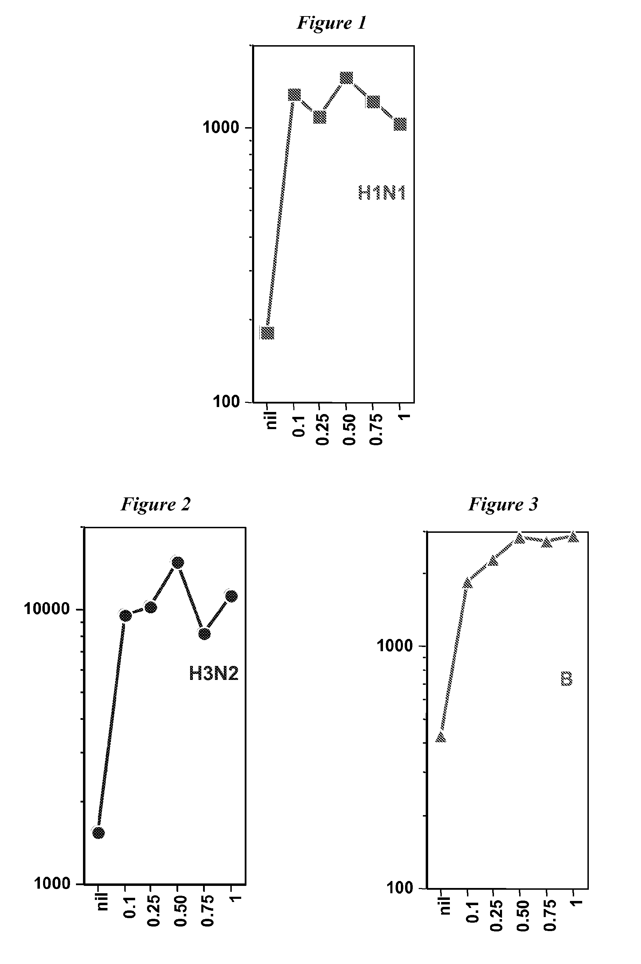

Figure 1 shows serum ELISA antibody titers (Log₁₀) against the H1N1 strain for compositions with prepared using different emulsion:antigen volume ratios. Figure 2 shows the same data for H3N2, and Figure 3 shows the same data for the influenza B virus strain.

### MODES FOR CARRYING OUT THE INVENTION

Influenza subunit vaccines were prepared from viruses grown on MDCK cell culture. The strains were: (i) A/Wyoming H3N2; (ii) A/New Caledonia H1N1; and (iii) B/Jiangsu. These vaccines were used to prepare adjuvanted vaccines for immunizing mice via the intramuscular route, using 0.2µg HA per strain per vaccine dose. The adjuvant in the vaccines was MF59. The adjuvant was mixed with aqueous HA antigen at different ratios. The mice received the same volume of material in each case, and the amount of aqueous HA antigen was constant for all experiments. However, the volume of MF59 was reduced from a maximum of 1:1. Volume ratios of 0.75, 0.50, 0.25 and 0.10 were used. control used no adjuvant.

As shown in Figure 1, reducing the amount of MF59 emulsion by up to ten times had no or little impact on overall immunogenicity. Thus the amount of an emulsion adjuvant required for an influenza vaccine can be reduced from the 1:1 ratio used in FLUAD^{TM}, thereby allowing more vaccines to be made from a given amount of emulsion.

It will be understood that the invention has been described by way of example only and modifications may be made whilst remaining within the scope and spirit of the invention.

### REFERENCES (the contents of which are hereby incorporated by reference)

[1] Vaccines. (eds. Plotkin & Orenstein). 4th edition, 2004, ISBN: 0-7216-9688-0.
[2] Minutello et al. (1999) Vaccine 17:99-104.
[3] Nicholson et al. (2001) Lancet 357:1937-43.
[4] Stepehenson et al. (2003) Vaccine 21:1687-93.
[5] WO90/14837.
[6] Podda & Del Giudice (2003) Expert Rev Vaccines 2:197-203.
[7] Podda (2001) Vaccine 19: 2673-2680.
*[8]* Vaccine Design: The Subunit and Adjuvant Approach (eds. Powell & Newman) Plenum Press 1995 (ISBN 0-306-44867-X).
*[9]* Vaccine Adjuvants: Preparation Methods and Research Protocols (Volume 42 of Methods in Molecular Medicine series). ISBN: 1-59259-083-7. Ed. O'Hagan.
[10] Allison & Byars (1992) Res Immunol 143:519-25.
[11] Hariharan et al. (1995) Cancer Res 55:3486-9.
[12] WO95/11700.
[13] US patent 6,080,725.
[14] W02005/097181.
[15] W02006/113373.
[16] Han et al. (2005) Impact of Vitamin E on Immune Function and Infectious Diseases in the Aged at Nutrition, Immune functions and Health EuroConference, Paris, 9-10 June 2005.
[17] US- 6630161.
[18] WO96/37624.
[19] WO98/46262.
[20] WO02/28422.
[21] WO02/067983.
[22] WO02/074336.
[23] WO02/097072.
[24] WO2005/113756.
[25] WO01/21151.
[26] Huckriede et al. (2003) Methods Enzymol 373:74-91.
[27] Herlocher et al. (2004) J Infect Dis 190(9):1627-30.
[28] Le et al. (2005) Nature 437(7062):1108.
[29] World Health Organisation (2005) Emerging Infectious Diseases 11(10):1515-21*.*
[30] Hoffmann et al. (2002) Vaccine 20:3165―3170.
[31] Subbarao et al. (2003) Virology 305:192―200.
[32] Liu et al. (2003) Virology 314:580―590.
[33] Ozaki et al. (2004) J. Virol. 78:1851-1857.
[34] Webby et al. (2004) Lancet 363:1099―1103*.*
[35] WO00/60050.
[36] WO01/04333.
[37] US 6649372.
[38] Neumann et al. (2005) Proc Natl Acad Sci USA 102:16825-9.
[39] W02006/067211.
[40] WO01/83794.
[41] Hoffmann et al. (2000) Virology 267(2):310-7.
[42] WO97/37000.
[43] Brands et al. (1999) Dev Biol Stand 98:93-100*.*
[44] Halperin et al. (2002) Vaccine 20:1240-7.
[45] Tree et al. (2001) Vaccine 19:3444-50.
[46] Kistner et al. (1998) Vaccine 16:960-8.
[47] Kistner et al. (1999) Dev Biol Stand 98:101-110.
[48] Bruhl et al. (2000) Vaccine 19:1149-58.
[49] Pau et al. (2001) Vaccine 19:2716-21.
[50] *http:*//*www.atcc.org*/
[51] *http:*//*locus.umdnj.edul*
[52] WO03/076601.
[53] WO2005/042728.
[54] WO03/043415.
[55] WO01/85938
[56] W02006/108846
[57] EP-A-1260581 (WO01/64846).
[58] W02006/071563.
[59] W02005/113758.
[60] W02006/027698.
[61] Lundblad (2001) Biotechnology and Applied Biochemistry 34:195-197.
[62] Guidance for Industry: Bioanalytical Method Validation. U.S. Department of Health and Human Services Food and Drug Administration Center for Drug Evaluation and Research (CDER) Center for Veterinary Medicine (CVM). May 2001.
[63] Ji et al. (2002) Biotechniques. 32:1162-7.
[64] Briggs (1991) JParenter Sci Technol. 45:7-12.
[65] Lahijani et al. (1998) Hum Gene Ther. 9:1173-80.
[66] Lokteff et al. (2001) Biologicals. 29:123-32.
[67] EP-B-0870508.
[68] US 5948410.
[69] International patent application entitled "CELL-DERIVED VIRAL VACCINES WITH LOW LEVELS OF RESIDUAL CELL DNA", filed 1st November 2006 claiming priority US-60/732786.
[70] WO03/023021
[71] WO03/023025
[72] WO97/37001.
[73] WO01/22992.
[74] Hehme *et al.* (2004) *Virus Res.* 103(1-2):163-71.
[75] Treanor et al. (1996) J Infect Dis 173:1467-70.
[76] Keitel et al. (1996) Clin Diagn Lab Immunol 3:507-10.
[77] Gennaro (2000) Remington: The Science and Practice of Pharmacy. 20th edition, ISBN: 0683306472.
[78] Banzhoff (2000) Immunology Letters 71:91-96.
[79] Nony et al. (2001) Vaccine 27:3645-51.
[80] W02005/089837.
[81] US 6,692,468.
[82] WO00/07647.
[83] WO99/17820.
[84] US 5,971,953.
[85] US 4,060,082.
[86] EP-A-0520618.
[87] WO98/01174.
[88] Potter & Oxford (1979) Br Med Bull 35: 69―75.
[89] Greenbaum et al. (2004) Vaccine 22:2566-77.
[90] Zurbriggen et al. (2003) Expert Rev Vaccines 2:295-304.
[91] Piascik (2003) J Am Pharm Assoc (Wash DC). 43:728-30.
[92] Mann et al. (2004) Vaccine 22:2425-9.
[93] Halperin et al. (1979) Am J Public Health 69:1247-50.
[94] Herbert et al. (1979) J lnfect Dis 140:234-8.
[95] Chen et al. (2003) Vaccine 21:2830-6.
[96] Powers & Belshe (1993) J Infect Dis 167:584-92.
[97] Mbawuike et al. (1996) Cell Immunol 173:64-78*.*
[98] Tassignon et al. (2005) J Immunol Meth 305:188-98.
[99] Myers et al. (1990) pages 145-156 of Cellular and molecular aspects of endotoxin reactions*.*
[100] Ulrich (2000) Chapter 16 (pages 273-282) of reference 9.
[101] Johnson et al. (1999) J Med Chem 42:4640-9.
[102] Baldrick et al. (2002) Regulatory Toxicol Pharmacol 35:398-413*.*
[103] US 4,680,338.
[104] US 4,988,815.
[105] WO92/15582.
[106] Stanley (2002) Clin Exp Dermatol 27:571-577.
[107] Wu et al. (2004) Antiviral Res. 64(2):79-83.
[108] Vasilakos et al. (2000) Cell Immunol. 204(1):64-74.
[109] US patents 4689338, 4929624, 5238944, 5266575, 5268376, 5346905, 5352784, 5389640, 5395937, 5482936, 5494916, 5525612, 6083505, 6440992, 6627640, 6656938, 6660735, 6660747, 6664260, 6664264, 6664265, 6667312, 6670372, 6677347, 6677348, 6677349, 6683088, 6703402, 6743920, 6800624, 6809203, 6888000 and 6924293.
[110] Jones (2003) Curr Opin Investig Drugs 4:214-218.
[111] W02004/060308.
[112] W02004/064759.
[113] US 6,924,271.
[114] US2005/0070556.
[115] US 5,658,731.
[116] US 5,011,828.
[117] W02004/87153.
[118] US 6,605,617.
[119] WO02/18383.
[120] WO2004/018455.
[121] WO03/082272.
[122] WO2006/002422..
[123] WO03/011223.
[124] Johnson et al. (1999) Bioorg Med Chem Lett 9:2273-2278.
[125] Evans et al. (2003) Expert Rev Vaccines 2:219-229.
[126] Thompson et al. (2005) JLeukoc Biol 78: 'The low-toxicity versions of LPS, MPL® adjuvant and RC529, are efficient adjuvants for CD4+ T cells'.
[127] Andrianov et al. (1998) Biomaterials 19:109-115*.*
[128] Payne et al. (1998) Adv Drug Delivery Review 31:185-196.
[129] Wong et al. (2003) J Clin Pharmacol 43(7):735-42.
[130] US2005/0215517.
[131] Thompson et al. (2003) Methods in Molecular Medicine 94:255-266.
[132] Kandimalla et al. (2003) Nucleic Acids Research 31:2393-2400.
[133] WO02/26757.
[134] WO99/62923.
[135] Krieg (2003) Nature Medicine 9:831-835.
[136] McCluskie et al. (2002) FEMS Immunology and Medical Microbiology 32:179-185.
[137] WO98/40100.
[138] US 6,207,646.
[139] US 6,239,116.
[140] US 6,429,199.
[141] Kandimalla et al. (2003) Biochemical Society Transactions 31 (part 3):654-658.
[142] Blackwell et al. (2003) J ImmunoI170:406l-4068*.*
[143] Krieg (2002) Trends Immunol 23:64-65.
[144] WO01/95935.
[145] Kandimalla et al. (2003) BBRC 306:948-953.
[146] Bhagat et al. (2003) BBRC 300:853-861.
[147] WO03/035836.
[148] WO01/22972.
[149] UK patent application GB-A-2220211.
[150] WO 94/21292.
[151] WO94/00153.
[152] WO95/17210.
[153] WO96/26741.
[154] WO93/19780.

## Claims

1. A process for making an adjuvanted influenza vaccine, comprising a step of mixing substantially equal volumes of (i) an oil-in-water emulsion and (ii) an aqueous preparation of an influenza virus antigen, wherein the concentration of hemagglutinin in component (ii) is more than 60µg per influenza virus strain per ml and (optionally) wherein the volume of a unit dose of the vaccine is less than 0.5ml.

2. A process for making an adjuvanted influenza vaccine, comprising a step of mixing substantially equal volumes of (i) an oil-in-water emulsion and (ii) an aqueous preparation of an influenza virus antigen, wherein the volume of a unit dose of the vaccine is less than 0.5ml; and (optionally) the concentration of hemagglutinin in component (ii) is about 60µg per influenza virus strain per ml.

3. A process for making an adjuvanted influenza vaccine, comprising the steps of: (a) mixing substantially equal volumes of (i) an oil-in-water emulsion and (ii) an aqueous preparation of an influenza virus antigen, to give a bulk vaccine; and (b) removing at least one unit dose from the bulk vaccine, where the volume of the unit dose is less than 0.5ml; and (optionally) the concentration of hemagglutinin in component (ii) is about 60µg per influenza virus strain per ml.

4. A process for making an adjuvanted influenza vaccine, comprising a step of mixing substantially equal volumes of (i) an oil-in-water emulsion and (ii) an aqueous preparation of an influenza virus antigen, wherein the concentration of hemagglutinin in the vaccine is less than 30µg per influenza virus strain per ml.

5. A process for making an adjuvanted influenza vaccine, comprising the step of mixing a first volume of an oil-in-water emulsion with a second volume of an aqueous preparation of an influenza virus antigen, wherein the second volume is greater than the first volume.

6. The process of claim 5, wherein the concentration of hemagglutinin in the second volume is about 60µg per influenza virus strain per ml or less than 60µg per influenza virus strain per ml.

7. A vaccine composition comprising an oil-in-water emulsion and influenza virus antigen, wherein the vaccine has a volume of less than 0.5ml.

8. A vaccine composition comprising influenza virus haemagglutinin and squalene, wherein the weight ratio of squalene to haemagglutinin is between 50 and 2000 and wherein the vaccine has a volume of less than 0.5ml.

9. The vaccine of claim 8, with a HA concentration of about 30µg per strain per ml, with a HA concentration of >30µg per strain per ml or a HA concentration of <30µg per strain per ml.

10. A composition comprising an oil in water emulsion and hemagglutinin from n influenza virus strains, wherein the weight ratio of oil to hemagglutinin is less than 640/n and (optionally) wherein the value of n is 1, 2, 3 or 4.

11. The process or composition of any preceding claim, wherein the oil-in-water emulsion includes squalene and/or polysorbate 80.

12. The process or composition of any preceding claim, wherein the influenza virus antigen is
(a) inactivated virus, for example wherein the influenza virus antigen comprises whole virus, split virus, or purified surface antigens; and/or
(b) from a H1, H2, H3, H5, H7 or H9 influenza A virus subtype; and/or
(c) prepared from an influenza virus grown on eggs.

13. The process or composition of any preceding claim, wherein the influenza virus antigen is prepared from an influenza virus grown on cell culture, for example a MDCK cell culture.

14. The process or composition of claim 13, wherein the composition is free from ovalbumin, ovomucoid and chicken DNA.

15. A kit for preparing the composition of any one of claims 7 to 14.
